# EUROPEAN PATENT APPLICATION

(11) **EP 0 982 387 A1**
(43) Date of publication of application: **01.03.2000**
(21) Application number: 98115937.9
(22) Date of filing: 24.08.1998
(51) Int. Cl.: C09K 19/04, C09K 19/30, C07C 25/24

(54) **Liquid crystal compound having negative dielectric anisotropy, liquid crystal composition containing said liquid crystal compound and liquid crystal display device using said composition**

(71) Applicant: Chisso Corporation, Osaka-shi Osaka 530-0055 (JP)
(72) Inventor: Yamada, Keizo, Chiba-shi, Chiba-ken (JP); Yano, Hitoshi, Ichihara-shi, Chiba-ken (JP)
(74) Representative: Müller-Boré & Partner Patentanwälte

(57) **Abstract**

A liquid crystal compound which has -C(=CH₂)CH₂- and/or -CH₂C(=CH₂)- groups and a ring represented by the following general formula (1) in the general structure of the compound: 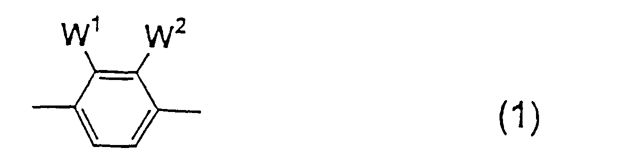 wherein W¹ and W² each independently represents a fluorine or chlorine atom, provided that each atom constituting the compound may be substituted with its isotope. The liquid crystal compound has a negative dielectric anisotropy value whose absolute value is large, a low viscosity, a controlled optical anisotropy value, a high specific resistance and a high voltage holding ratio and is stable against heat and irradiation with ultraviolet rays.

## Description

The present invention mainly relates to a novel liquid crystal compound having characteristic properties favorable for a liquid crystal composition used for liquid crystal display devices of various display modes such as vertically aligned mode, IPS (in-plane switching) mode, TFT (thin film transistor) mode, TN (twisted nematic) mode or STN (super twisted nematic) mode, in particular, vertically aligned mode and IPS display modes; a liquid crystal composition using the liquid crystal compound and having excellent physical properties; and a liquid crystal display device produced using the liquid crystal composition. In the present invention, the term "liquid crystal compound" is a general term for compounds capable of exhibiting liquid crystal phases and compounds which do not exhibit any liquid crystal phase, but are useful as ingredients for liquid crystal compositions.

Up to this time, there have been proposed various liquid crystal display modes. As an example thereof, there can be listed the following display mode (see "Up-To-Date Liquid Crystal Technique", edited by Kogyochosa Kai, 1983). Examples of modes which make use of liquid crystal compositions having positive dielectric anisotropy are TN modes, STN modes, or TN based AM (active matrix) modes (such as TFT mode or MIM (metal-insulating film-metal) mode). Moreover, as modes which make use of liquid crystal compositions having negative dielectric anisotropy, there may be listed, for instance, ECB (Electric field-control birefringent effect) modes (such as HAN (hybrid molecular arrangement) mode or DAP (vertical molecular arrangement) mode), DS (dynamic scattering) modes, GH (guest-host) type modes or PC (phase transition) modes.

Among these modes, the mainstreams thereof which have presently been used are those which make use of liquid crystal compositions having positive dielectric anisotropy. Contrary to this, there has been a delay in the practical application of the modes utilizing liquid crystal compositions having negative dielectric anisotropy. In connection with this, the development of liquid crystal compositions having negative dielectric anisotropy and compounds per se used in the compositions have also been insufficient as compared with the development of such compositions having positive dielectric anisotropy and compounds used in these compositions.

Under these circumstances, there have recently been reported wide variety of attempts, in particular, to eliminate one of disadvantages of the liquid crystal display techniques, i.e., the narrowness of the viewing angle. An example thereof is IPS mode (see, for instance, R. Kiefer et al., JAPAN DISPLAY '92, 547 (1992); M. Oh-e et al., ASIA DISPLAY '95, 577 (1995); TOKUHYO Hei 5-505247; Japanese Un-Examined Patent Publication (hereinafter referred to as "J.P. KOKAI") No. Hei 7-128647). One of the characteristic properties of IPS is that a comb-like electrode is arranged only on one substrate in the liquid crystal panel of this type, while electrodes are formed on the both upper and lower substrates in the conventional liquid crystal panel. Another characteristic property of the mode is that a liquid crystal composition can be used irrespective of the dielectric anisotropy of the composition (positive or negative).

As another example of the attempts to extend the viewing angle, there may be listed a mode wherein the viewing angle is improved by making use of the vertical alignment of liquid crystal molecules (see, for instance, J.P. KOKAI No. Hei 2-176625). One of the characteristic properties of this mode is to use a liquid crystal composition having negative dielectric anisotropy.

With these points as background, it has intensively been required for the development of a liquid crystal compound having negative dielectric anisotropy and a liquid crystal composition.

Incidentally, in all of the display modes, the liquid crystal compositions used therein should have not only an appropriate dielectric anisotropy value, but also the best-controlled other characteristic properties such as values of optical anisotropy (Δn), elastic constant ratio: K₃₃/K₁₁ (K₃₃: bend elastic constant; K₁₁: spray elastic constant) and further they must satisfy such requirements that the temperature of the liquid crystal phase falls within an appropriate range and that they should have a low viscosity even at a low temperature.

None of conventionally known liquid crystal compounds satisfy all of these requirements when they are used alone. For this reason, a composition which can be used as a material for exhibiting a liquid crystal phase has usually been prepared by mixing several kinds to twenty or so kinds of compounds capable of exhibiting liquid crystal phases and optionally several kinds of compounds incapable of forming any liquid crystal phase. Therefore, each liquid crystal compound should also satisfy such requirements that it has good miscibility with other liquid crystal compounds and that it also has good miscibility therewith in a low temperature range since the composition is also used in low temperature environments.

As has been described above, however, it has been the mainstream to use liquid crystal compositions comprising liquid crystal compounds having positive dielectric anisotropy in the conventional display modes and the development of liquid crystal compositions and compounds having negative dielectric anisotropy have still been insufficient. Therefore, the conventional liquid crystal compounds and compositions have not been able to completely cope with such diversified modes and related various characteristics. For instance, they suffer from the following various problems:
. They have negative dielectric anisotropy, but the absolute value thereof is small;
. They do not permit the reduction of the driving voltage of the display devices because of their large elastic constants;
. They cannot be used in a large amount because of poor miscibility with other ingredients for the liquid crystal compositions and display devices using them;
. It is difficult to freely establish the optical anisotropy of these compositions or compounds;
. They have high viscosities; and
. They are physically and chemically less stable.

An example of the conventionally known liquid crystal compounds having negative dielectric anisotropy is a liquid crystal compound having a 2,3-difluorophenylene group (J.P. KOKAI No. Sho 57-114532). However, the absolute values of the negative dielectric anisotropy of these compounds are not always sufficiently large and the compounds do not have desired characteristic properties such as a low elastic constant, good miscibility with other ingredients, a high degree in the design of optical anisotropy, a low viscosity and high physicochemical stability. For this reason, there has been desired for further improvement of these compounds and compositions.

In addition, there has not been reported any investigation to improve the foregoing characteristic properties of the compound having a 2,3-difluorophenylene group, for instance, any satisfactory attempt to increase the absolute value of the negative dielectric anisotropy thereof. In particular, there has not been proposed such an idea that a group such as -C(=CH₂)CH₂- or -CH₂C(=CH₂)- is introduced into the compound having a 2,3-difluorophenylene group.

As has been described above, the compounds of the present invention cannot easily be hit upon on the basis of the teachings or knowledges of the prior art.

Accordingly, an object of the present invention is to provide a novel liquid crystal compound which can be not only used in various display modes utilizing compounds or compositions having negative dielectric anisotropy, such as vertically aligned mode as disclosed in J.P. KOKAI No. Hei 2-176625, IPS mode, ECB modes (such as HAN mode or DAP mode), DS mode, GH mode or PC mode, but also used for controlling various characteristics of liquid crystal compositions for various display modes utilizing compounds or compositions having positive dielectric anisotropy, such as TN mode, STN mode, or TN based AM (TFT or MIM) mode, and which can solve the foregoing problems, as well as a liquid crystal composition containing the compound and a liquid crystal display device produced using the composition.

The inventors of this invention have conducted various studies to accomplish the foregoing objects, as a result, have found that the compounds as specified in the following first aspect and related embodiments of the present invention not only have negative dielectric anisotropy whose absolute values are large, but also have appropriate optical anisotropy values, small elastic constants, good miscibility with other substances, low viscosities and high physicochemical stability, that the use of the foregoing compounds permits the formation of liquid crystal compositions whose elastic constant is small, whose dielectric and optical anisotropy values can appropriately be established, which have low viscosities and excellent physicochemical stability and, in particular, which have negative dielectric anisotropy whose absolute values are large and that an excellent liquid crystal display device can be obtained by the use of the composition and thus have completed the present invention. According to a first aspect of the present invention, there is provided a liquid crystal compound having C(=CH₂)CH₂- and/or -CH₂C(=CH₂)- groups and a ring represented by the following general formula (1) in the general structure of the compound: 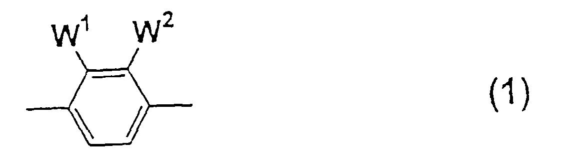 wherein W¹ and W² each independently represents a fluorine or chlorine atom, provided that each atom constituting the compound may be substituted with its isotope.

The compound is preferably those represented by the following general formula (2):

R¹-(A¹-X¹)ₐ₁-(A²-X²)ₐ₂-(A³-X³)ₐ₃-(A⁴-X⁴)ₐ₄-(A⁵)ₐ₅-Y¹ (2)

wherein R¹ and Y¹ each independently represents an alkyl group having 1 to 20 carbon atoms, a hydrogen atom, a halogen atom, a cyano group, a cyanate residue, an isocyano group or an isothiocyanate residue, provided that at least one of the methylene groups present in the alkyl group, which are not adjacent to one another, may be substituted with an oxygen, sulfur or nitrogen atom, -C ≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom or that these alkyl groups may be optically active ones; X¹, X², X³ and X⁴ each independently represents a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡C-, -C=CCH=CH-, -C(=CH₂)CH₂- or -CH₂C(=CH₂)-, provided that at least one of the substituents X¹, X², X³ and X⁴ represent a group -C(=CH₂)CH₂- or -CH₂C(=CH₂)-; the rings A¹, A², A³, A⁴ and A⁵ each independently represents a trans-cyclohexane-1,4-diyl, cyclohexane-1-en-1,4-diyl, 1,4-phenylene, bicyclo[1.1.1] pentane-1,3-diyl group, or a ring represented by the foregoing formula (1): 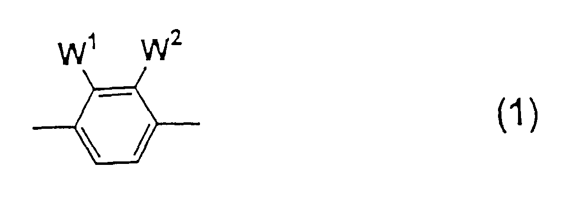 wherein W¹ and W² each independently represents a fluorine or chlorine atom, provided that at least one of these rings A¹, A², A³, A⁴ and A⁵ represent a ring represented by Formula (1), that the carbon atoms constituting these rings may be substituted with a nitrogen, oxygen or sulfur atom and that each hydrogen atom present on the rings may be substituted with a halogen atom or a cyano group; a¹, a², a³, a⁴ and a⁵ each independently represents 0 or 1, provided that they satisfy the relation: a¹+a²+a³+a⁴+a⁵≧1; and each atom constituting the compound may be substituted with its isotope.

More preferably, the group represented by Formula (1) is 2,3-difluorobenzene-1,4-diyl.

According to a second aspect of the present invention, there is provided a liquid crystal composition which comprises at least two components including at least one liquid crystal compound defined above.

According to a third aspect of the present invention, there is provided a liquid crystal composition which comprises at least one liquid crystal compound defined above as a first component and at least one compound selected from the group consisting of those represented by the following general formulas (3), (4) and (5): 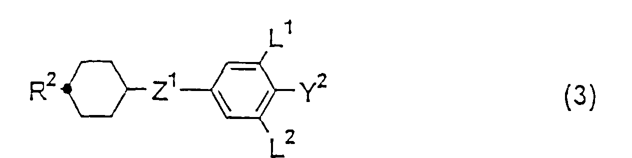 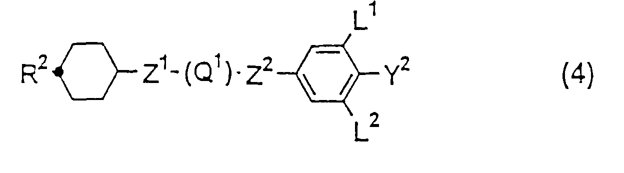 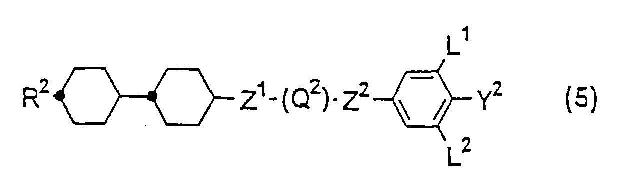 wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom, -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents -(CH₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-1,4-diyl group, or a 1,4-phenylene group whose hydrogen atom may be substituted with a fluorine atom; and each atom constituting the compound may be substituted with its isotope.

According to a fourth aspect of the present invention, there is provided a liquid crystal composition which comprises at least one liquid crystal compound defined above as a first component and at least one compound selected from the group consisting of those represented by the following general formulas (6) and (7) as a second component: 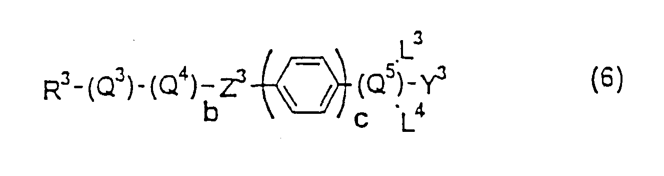 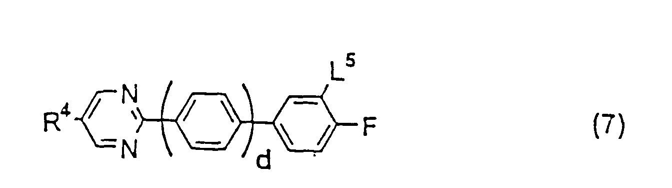 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cyano group or -C≡C-CN; the ring Q³ represents a trans-cyclohexane-1,4-diyl, 1,4-phenylene, 1,3-dioxane-2,5-diyl or pyrimidine-2,5-diyl group; the ring Q₄ represents a trans-cyclohexane-1,4-diyl or pyrimidine-2,5-diyl group, or a 1,4-phenylene group whose hydrogen atom may be substituted with a fluorine atom; the ring Q⁵ represents a trans-cyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

According to a fifth aspect of the present invention, there is provided a liquid crystal composition which comprises at least one liquid crystal compound defined above as a first component; at least one compound selected from the group consisting of those represented by the foregoing general formulas (3), (4) and (5) as a second component; and at least one compound selected from the group consisting of those represented by the following general formulas (8), (9) and (10) as a third component:

R⁵·(Q⁶)-Z⁴·(Q⁷)-Z⁵―R⁶ (8)

R⁵·(Q⁶)-Z⁴·(Q⁷)-Z⁵-(Q⁸)·R⁶ (9)

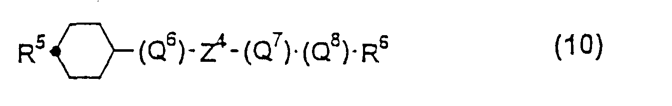

wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1,4-phenylene group whose hydrogen atom may be substituted with a fluorine atom; Z⁴ and Z⁵ each independently represents -C≡C-, -COO-, -(CH₂)₂-, -CH=CH- or a single bond; and each atom constituting the compound may be substituted with its isotope.

According to a sixth aspect of the present invention, there is provided a liquid crystal composition which comprises at least one liquid crystal compound defined above as a first component and at least one compound selected from the group consisting of those represented by the following general formulas (11), (12) and (13) as a second component: 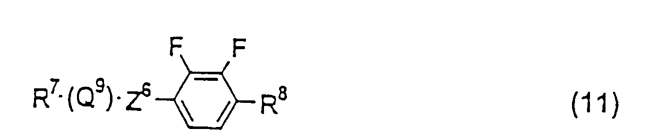 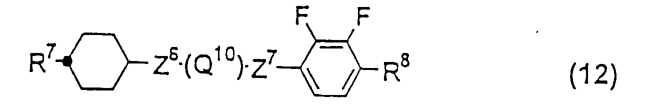 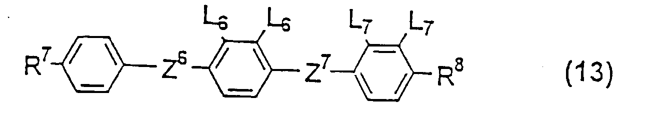 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁶ and L⁷ do not simultaneously represent a hydrogen atom; Z⁶ and Z⁷ each independently represents -(CH₂)₂-, -COO- or a single bond; and each atom constituting the compound may be substituted with its isotope.

According to a seventh aspect of the present invention, there is provided a liquid crystal composition which comprises at least one liquid crystal compound defined above as a first component; at least one compound selected from the group consisting of those represented by the foregoing general formulas (8), (9) and (10) as a second component; and at least one compound selected from the group consisting of those represented by the foregoing general formulas (11), (12) and (13) as a third component.

According to an eighth aspect of the present invention, there is provided a liquid crystal composition which comprises at least one liquid crystal compound defined above as a first component; at least one compound selected from the group consisting of those represented by the foregoing general formulas (6) and (7) as a second component; and at least one compound selected from the group consisting of those represented by the foregoing general formulas (8), (9) and (10) as a third component.

According to a ninth aspect of the present invention, there is provided a liquid crystal composition which comprises at least one liquid crystal compound defined above as a first component; at least one compound selected from the group consisting of those represented by the foregoing general formulas (3), (4) and (5) as a second component; at least one compound selected from the group consisting of those represented by the foregoing general formulas (6) and (7) as a third component; and at least one compound selected from the group consisting of those represented by the foregoing general formulas (8), (9) and (10) as a fourth component.

According to a tenth aspect of the present invention, there is provided a liquid crystal composition which comprises at least one optically active compound in addition to one of the liquid crystal compositions according to the second to ninth aspects of the present invention.

According to an eleventh aspect of the present invention, there is provided a liquid crystal display device constituted by the use of one of the liquid crystal compositions according to the second to tenth aspects of the present invention.

The sixth and seventh aspects of the present invention relate to N-type (having negative Δ ε) liquid crystal compositions. The N-type liquid crystal composition can be driven according to a variety of display modes such as vertically aligned mode and IPS mode as disclosed in J.P. KOKAI No. Hei 2-176625.

Moreover, the present inventions according to the third, fourth, fifth, eighth and ninth aspects of the present invention relate to P-type (having positive Δ ε) liquid crystal compositions, but an N-type compound may also be used as a component for such P-type liquid crystal compositions. This permits not only arbitrary establishment of the dielectric anisotropy value of the liquid crystal composition depending on any particular use, but also the control of other characteristic properties of the composition such as the optical anisotropy value and the elastic constant.

Preferred embodiments of the compounds of the present invention, which carry -C(=CH₂)CH₂- and/or -CH₂C(=CH₂)- groups and the rings of Formula (1) in the skeletal structures thereof will now be detailed below.

In the ring represented by Formula (1), W¹ and W² each independently represents a fluorine or chlorine atom.

Specific examples of the rings represented by Formula (1) are as follows: 2,3-difluorobenzene-1,4-diyl, 2,3-dichlorobenzene-1,4-diyl, and 2-chloro-3-fluorobenzene-1,4-diyl rings.

Preferred embodiments of the compounds of the present invention, represented by Formula (2) will be described below.

The substituents R¹ and Y¹ of the compound of Formula (2) each independently represents an alkyl group having 1 to 20 carbon atoms, a hydrogen or halogen atom, a cyano or isocyano group, or a cyanate or isothiocyanate residue, provided that at least one of the methylene groups present in the alkyl group, which are not adjacent to each other, may be substituted with an oxygen, sulfur or nitrogen atom, a group -C ≡C-, a silylene group in which at least one hydrogen atom may be substituted with an alkyl group having 1 to 10 carbon atoms, or a vinylene group, that at least one of the hydrogen atoms present in the alkyl group may be substituted with a fluorine or chlorine atom or that these alkyl groups may be optically active ones.

Specific examples of the alkyl group represented by R¹ and Y¹ in Formula (2) include saturated alkyl groups simply consisting of carbon and hydrogen atoms, haloalkyl groups, alkoxy or haloalkoxy groups, alkenyl or haloalkenyl groups, alkenyloxy or haloalkenyloxy groups, alkynyl or haloalkynyl groups, alkynyloxy or haloalkynyloxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, and alkoxycarbonyl or haloalkoxycarbonyl groups.

Specific examples of R¹ and Y¹ are groups or atoms described below:
-CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -CFH₂, -CF₂H, -CF₃, -CH₂CH₂F, -CH₂CHF₂, -CH₂CF₃, -CF₂CH₃, -CF₂CF₂H, -CHFCF₃, -CF₂CF₃, -(CH₂)₂CH₂F, -(CH₂)₂CHF₂, -(CH₂)₂CF₃, -CH₂CF₂CH₃, -CH₂CF₂CF₃, -CF₂C₂H₅, -C₃F₇, -CF₂CFHCF₃, -(CH₂)₃CH₂F, -(CH₂)₄CH₂F, -(CH₂)₅CH₂F, -CF₂C₃H₇, -CH₂CF₂C₂H₅, -(CH₂)₂CF₂CH₃, -CF₂C₄H₉, -CH₂CF₂CH₃, -(CH₂)₂CF₂C₂H₅, -(CH₂)₃CF₂CH₃, -OCH₃, -OC₂H₅, -OC₃H₇, -OC₄H₉, -OC₅H₁₁, -OC₆H₁₃, -OC₇H₁₅, -OC₈H₁₇, -OC₉H₁₉, -OC₁₀H₂₁, -OCFH₂, -OCF₂H, -OCF₃, -OCH₂CH₂F, -OCH₂CHF₂, -OCH₂CF₃, -OCF₂CH₃, -OCF₂CF₂H, -OCHFCF₃, -OCF₂CF₃, O(CH₂)₂CH₂F, -O(CH₂)₂CHF₂, -O(CH₂)₂CF₃, -OCH₂CF₂CH₃, -OCH₂CF₂CF₃, -OCF₂C₂H₅, -OC₃F₇, -OCF₂CFHCF₃, -O(CH₂)₃CH₂F, -O(CH₂)₄CH₂F, -O(CH₂)₅CH₂F, -OCF₂C₃H₇, -OCH₂CF₂C₂H₅, -O(CH₂)₂CF₂CH₃, -OCF₂C₄H₉, -OCH₂CF₂CH₃, -O(CH₂)₂CF₂C₂H₅, -O(CH₂)₃CF₂CH₃, -CH=CH₂, -CH=CHCH₃, -CH=CHC₂H₅, -CH=CHC₃H₇, -CH=CHC₄H₉, -CH₂CH=CHCH₃, -CH₂CH=CHC₂H₅, -CH₂CH=CHC₃H₇, -CH₂CH=CHC₄H₉, -(CH₂)₂CH=CHCH₃, -(CH₂)₂CH=CHC₂H₅, -(CH₂)₂CH=CHC₃H₇, -(CH₂)₂CH=CHC₄H₉, -CH₂CH=CH₂, -(CH₂)₂CH=CH₂, -(CH₂)₃CH=CH₂, -(CH₂)₄CH=CH₂, -(CH₂)₅CH=CH₂, -CH=CHCH=CH₂, -CH=CHCH₂CH=CH₂, -CH=CH(CH₂)₂CH=CH₂, -CH=CH(CH₂)₃CH=CH₂, -CH₂CH=CH(CH₂)₂CH=CH₂, -(CH₂)₂CH=CH(CH₂)₂CH=CH₂, -CF=CF₂, -CH=CF₂, -CH=CHF, -CH₂CF=CF₂, -CH₂CH=CHF, -(CH₂)₂CH=CF₂, -(CH₂)₂CH=CHF, -(CH₂)₃CH=CF₂, -(CH₂)₃CH=CHF, -(CH₂)₄CH=CF₂, -(CH₂)₄CH=CHF, -(CH₂)₅CH=CF₂, -(CH₂)₅CH=CHF, -CH=CHCH₂CH₂F, -CH=CHCH₂CH₂Cl, -CH=CHCH₂CHF₂, -CH=CHCH₂CF₃, -CH=CH(CH₂)₂CH₂F, -CH=CH(CH₂)₂CHF₂, -CH=CH(CH₂)₂CF₃, -CH=CH(CH₂)₃CH₂F, -CH=CH(CH₂)₃CHF₂, -CH=CH(CH₂)₃CF₃, -CH=CH(CH₂)₄CH₂F, -CH=CH(CH₂)₄CHF₂, -CH=CH(CH₂)₄CF₃, -CH₂CH=CH(CH₂)₂CH₂F, -CH₂CH=CHCH₂CH₂F, -(CH₂)₂CH=CH(CH₂)₂CH₂F, -(CH₂)₂CH=CHCH₂CH₂F, -CH=CHCH₂CH=CF₂, -CH=CH(CH₂)₂CH=CF₂, -CH=CH(CH₂)₂CF=CF₂, -CH=CH(CH₂)₃CHFCH₃, -OCH₂CH=CHCH₃, -OCH₂CH=CHC₂H₅, -OCH₂CH=CHC₃H₇, -OCH₂CH=CHC₄H₉ -O(CH₂)₂CH=CHCH₃, -O(CH₂)₂CH=CHC₂H₅, -O(CH₂)₂CH=CHC₃H₇, -O(CH₂)₂CH=CHC₄H₉, -OCH₂CH=CH₂, -O(CH₂)₂CH=CH₂, -O(CH₂)₃CH=CH₂, -O(CH₂)₄CH=CH₂, -O(CH₂)₅CH=CH₂, -OCH₂CH=CH(CH₂)₂CH=CH₂, -O(CH₂)₂CH=CH(CH₂)₂CH=CH₂, -OCH₂CF=CF₂, -OCH₂CH=CF₂, -OCH₂CF=CHF, -O(CH₂)₂CH=CF₂, -O(CH₂)₂CH=CHF, -O(CH₂)₃CH=CF₂, -O(CH₂)₃CH=CHF, -O(CH₂)₄CH=CF₂, -O(CH₂)₄CH=CHF, -O(CH₂)₅CH=CF₂, -O(CH₂)₅CH=CHF, -OCH₂CH=CHCH₂CH₂F, -O(CH₂)₂CH=CH(CH₂)₂CH₂F, -O(CH₂)₂CH=CHCH₂CH₂F, -C≡CH, -C≡CCH₃, -C≡ CC₂H₅, -C ≡CC₃H₇, -C ≡CC₄H₉, -CH₂C≡CH, -CH₂C ≡CCH₃, -CH₂C ≡CC₂H₅, -CH₂C≡CC₃H₇, -CH₂C≡CC₄H₉, -(CH₂)₂C ≡CH, -(CH₂)₂C=CCH₃, -(CH₂)₂C≡ CC₂H₅, -(CH₂)₂C ≡CC₃H₇, -(CH₂)₂C ≡CC₄H₉, -(CH₂)₃C ≡CH, -(CH₂)₃C≡ CCH₃, -(CH₂)₃C≡CC₂H₅, -(CH₂)₃C ≡CC₃H₇, -(CH₂)₃C ≡CC₄H₉, -C ≡CCF₃, -C≡CC₂F₅, -C ≡CC₃F₇, -CH₂C≡CCF₃, -(CH₂)₂C≡CCF₃, -OCH₂C≡CH, -OCH₂C ≡CCH₃, -OCH₂C≡CC₂H₅, -OCH₂C ≡CC₃H₇, -OCH₂C ≡CC₄H₉, -O(CH₂)₂C≡CH, -O(CH₂)₂C ≡CCH₃, -O(CH₂)₂C ≡CC₂H₅, -O(CH₂)₂C≡CC₃H₇, -O(CH₂)₂C≡ CC₄H₉, -O(CH₂)₃C≡CH, -O(CH₂)₃C ≡CCH₃, -O(CH₂)₃C ≡CC₂H₅, -O(CH₂)₃C≡ CC₃H₇, -O(CH₂)₃C≡CC₄H₉, -OCH₂C ≡CCF₃, -O(CH₂)₂C ≡CCF₃, -CH₂OCH₃, -CH₂OC₂H₅, -CH₂OC₃H₇, -CH₂OC₄H₉, -CH₂OC₅H₁₁, -(CH₂)₂OCH₃, -(CH₂)₂OC₂H₅, -(CH₂)₂OC₃H₇, -(CH₂)₂OC₄H₉, -(CH₂)₂OC₅H₁₁, -(CH₂)₃OCH₃, -(CH₂)₃OC₂H₅, -(CH₂)₃OC₃H₇, -(CH₂)₃OC₄H₉, -(CH₂)₃OC₅H₁₁, -(CH₂)₄OCH₃, -CF₂OCH₃, -CF₂OC₂H₅, -CF₂OC₃H₇, -CF₂OC₄H₉, -CF₂OC₅H₁₁, -CH₂OCF₃, -CH₂OC₂F₅, -OCH₂OCH₃, -OCH₂OC₂H₅, -OCH₂OC₃H₇, -OCH₂OC₄H₉, -OCH₂OC₅H₁₁, -O(CH₂)₂OCH₃, -O(CH₂)₂OC₂H₅, -O(CH₂)₂OC₃H₇, -O(CH₂)₂OC₄H₉, -O(CH₂)₂OC₅H₁₁, -O(CH₂)₃OCH₃, -O(CH₂)₃OC₂H₅, -O(CH₂)₃OC₃H₇, -O(CH₂)₃OC₄H₉, -O(CH₂)₃OC₅H₁₁, -O(CH₂)₄OCH₃, -OCH₂OCF₃, -OCH₂OC₂F₅, -COCF₃, -COCHF₂, -COC₂F₅, -COC₃F₇, -COCH₂CF₃, -OCOCH₃, -OCOC₂H₅, -OCOC₃H₇, -OCOC₅H₁₁, -OCOC₇H₁₅, -OCOCF₃, -OCOCHF₂, -OCOC₂F₅, -OCOC₃F₇, -OCOCH₂CF₃, -COOCH₃, -COOC₂H₅, -COOC₃H₇, -COOC₄H₉, -COOC₅H₁₁, -COOCH(CH₃)C₆H₁₃, -COOCH₂F, -COOCH₂CF₃, -COO(CH₂)₂CF₃, -COO(CH₂)₃CF₃, -COO(CH₂)₄CH₂F, hydrogen or halogen atoms, cyano or isocyano groups, and cyanate or isothiocyanate residues.

If paying attention, in particular, to the physicochemical stability of the compound of Formula (2), R¹ and Y¹ are preferably saturated alkyl groups simply consisting of carbon and hydrogen atoms, haloalkyl groups, alkoxy or haloalkoxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, alkoxycarbonyl or haloalkoxycarbonyl groups, hydrogen or halogen atoms, cyano or isocyano groups, and cyanate residues.

If it is desired to further increase the absolute value of the negative dielectric anisotropy of the compound of Formula (2), R¹ and Y¹ are preferably substituents having a low polarity such as saturated alkyl groups simply consisting of carbon and hydrogen atoms, alkoxy groups, alkenyl groups, alkenyloxy groups, alkynyl groups, alkynyloxy groups, alkoxyalkyl groups, alkoxyalkoxy groups, alkanoyl groups, alkanoyloxy groups, alkoxycarbonyl groups, and hydrogen atom.

At least one of the substituents X¹, X², X³ and X⁴ present on the compound of Formula (2) is the foregoing group: -C(=CH₂)CH₂- or -CH₂C(=CH₂)-.

Preferred substituents X¹, X², X³ and X⁴ other than the foregoing are as follows: a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡ C-, and -C≡CCH=CH-.

If paying attention, in particular, to the physicochemical stability of the compound of Formula (2), preferred substituents X¹, X², X³ and X⁴ are -C(=CH₂)CH₂- and -CH₂C(=CH₂)-, but other preferred examples of the substituents X¹, X², X³ and X⁴ are as follows:
Single bond, -(CH₂)₂-, -COO-, -OCO-, -CH₂O-, -OCH₂-, -(CH₂)₄-, -(CH₂)₃O-, -O(CH₂)₃-, -CF₂O-, -OCF₂-, -CH₂CF₂-, -CF₂CH₂-, -(CF₂)₂-, -(CF₂)₄-, -(CH₂)₂COO-, and -OCO(CH₂)₂-.

If it is desired to further increase the absolute value of the negative dielectric anisotropy of the compound of Formula (2), preferred substituents X¹, X², X³ and X⁴ are -C(=CH₂)CH₂- or -CH₂C(=CH₂)-, but other preferred examples thereof are as follows:
Single bond, -(CH₂)₂-, -COO-, -OCO-, -CH₂O-, -OCH₂-, -(CH₂)₄-, -(CH₂)₃O-, -O(CH₂)₃-, -CF₂O-, -OCF₂-, -CH₂CF₂-, -CF₂CH₂-, -(CF₂)₂-, -(CF₂)₄-, -(CH₂)₂COO-, and -OCO(CH₂)₂-.

At least one of the rings A¹, A², A³, A⁴ and A⁵ of the compound represented by Formula (2) is a ring represented by Formula (1). Specific examples thereof are those listed below:
2,3-Difluorobenzene-1,4-diyl, 2,3-dichlorobenzene-1,4-diyl, and 2-chloro-3-fluorobenzene-1,4-diyl.

Preferred rings other than the foregoing are, for instance, bicyclo[l.l.l]pentane-1,3-diyl, trans-cyclohexane-1,4-diyl, cyclohexa-l-ene-1,4-diyl and 1,4-phenylene, provided that the carbon atoms constituting these rings may be substituted with a nitrogen, oxygen or sulfur atom and that the hydrogen atoms present on the rings may likewise be substituted with a halogen atom or a cyano group.

Specific examples thereof include 1-cyano-trans-cyclohexane-1, 4-diyl, 2,2-difluoro-trans-cyclohexane-1,4-diyl, 2-fluorocyclohexa -1-ene-1,4-diyl, and 2,2-dichlorobicyclo[1.1.1]pentane-1, 3-diyl.

Examples of the rings whose hydrogen atoms present thereon may be substituted with a fluorine or chlorine atom or a cyano group are as follows:

Bicyclo[l.l.l]pentane-1,3-diyl, trans-cyclohexane-1,4-diyl, cyclohexa-l-ene-1,4-diyl, 1,4-phenylene, 1,3-dioxane-2,5-diyl, pyrimidine-2,5-diyl, pyridine-2,5-diyl, pyrazine-2,5-diyl, pyridazine-3,6-diyl, 1,3-dithian-2,5-diyl, 1,3-oxathian-2,5-diyl, 2,3-difluorobenzene-1,4-diyl and bicyclo[2.2.2]octane-1,4-diyl.

If paying attention, in particular, to the physicochemical stability of the compound of Formula (2), preferred rings A¹, A², A³, A⁴ and A⁵ of the compound represented by Formula (2) include those represented by Formula (1) (such as 2,3-difluorobenzene-1,4-diyl), but preferred rings A¹, A², A³, A⁴ and A⁵ other than the foregoing are as follows:

1-cyano-trans-cyclohexane-1,4-diyl, 2,2-difluoro-transcyclohexane-1,4-diyl, 2-fluorocyclohexa-l-ene-1,4-diyl, and 2,2-dichlorobicyclo[1.1.1]pentane-1,3-diyl.

Examples of the rings whose hydrogen atoms present thereon may be substituted with a fluorine or chlorine atom or a cyano group are bicyclo[1.1.1]pentane-1,3-diyl, trans-cyclohexane-1,4-diyl, cyclohexa-1-ene-1,4-diyl, 1,4-phenylene, 1,3-dioxane-2,5-diyl, pyrinidine-2,5-diyl, pyridine-2,5-diyl, pyridazine-3,6-diyl, 1,3-dithian-2,5-diyl, 2,3-difluorobenzene-1,4-diyl, and 1,3-oxathian-2,5-diyl.

If it is desired to further increase the absolute value of the negative dielectric anisotropy of the compound of Formula (2), preferred rings A¹, A², A³, A⁴ and A⁵ are those represented by Formula (1) (such as 2,3-difluorobenzene-1,4-diyl), but preferred rings A¹, A², A³, A⁴ and A⁵ other than those listed above are 1-cyano-trans-cyclohexane-1,4-diyl, 2,2-difluoro-trans-cyclohexane-1,4-diyl, 2-fluorocyclohexa -1-ene-1,4-diyl, and 2,2-dichlorobicyclo[1.1.1]pentane-1,3-diyl.

Examples of the rings whose hydrogen atoms present thereon may be substituted with a fluorine or chlorine atom or a cyano group include trans-cyclohexane-1,4-diyl, cyclohexa-1-ene-1,4-diyl, and pyridazine-3,6-diyl.

The compounds according to the first aspect of the present invention and preferred embodiments thereof have negative dielectric anisotropy values whose absolute values are large. Therefore, a liquid crystal composition having a negative dielectric anisotropy value can be prepared by mainly using the foregoing compounds. This liquid crystal composition is useful in the preparation of devices which make use of a liquid crystal composition having negative dielectric anisotropy, including, for instance, vertically aligned mode and IPS mode as disclosed in J.P. KOKAI No. Hei 2-176625. Moreover, the dielectric anisotropy value can appropriately be established by the use of a mixture of the compound of the present invention with other liquid crystal compounds or compositions to thus expand the area of applications of these other liquid crystal compounds or compositions.

The compounds according to the first aspect of the present invention and preferred embodiments thereof have small elastic constants and the temperature-dependency of the constants is also low. Therefore, the use of the compounds of the present invention would permit the preparation of novel liquid crystal compositions for a low driving voltage.

The compounds according to the first aspect of the present invention and preferred embodiments thereof exhibit good miscibility with other liquid crystal compounds. For this reason, if the compounds of the present invention are incorporated into liquid crystal compositions, any problem such as separation of compounds rarely arises. In addition, a large amount of the compound of the present invention can be incorporated into a liquid crystal composition and accordingly, the excellent characteristics of the compound of the present invention can intensively be reflected in the resulting liquid crystal composition.

The compounds according to the first aspect of the present invention and preferred embodiments thereof permit appropriate design of the structure of the compounds such that they have a desired level of the optical anisotropy value. In other words, the compound should be designed so as to have rings including a large number of sites having resonance structures such as aromatic rings, if it is necessary to use a compound having a particularly high optical anisotropy value. On the other hand, if it is necessary to use a compound having a low optical anisotropy value, the compound should be designed so as to have rings including a large number of sites free of any resonance structure such as trans-cyclohexane-1,4-diyl ring.

If describing this more fully, the compound of Formula (2) according to the present invention may have a desired level of the optical anisotropy value by appropriately selecting the substituents R¹, Y¹, X¹, X², X³, X⁴, ring A¹, ring A², ring A³, ring A⁴, ring A⁵, a¹, a², a³, a⁴ and a⁵. More specifically, the optical anisotropy value may be controlled by appropriately selecting the rings A¹, A², A³, A⁴ and A⁵. In other words, the selection of a ring or rings each including a large number of sites having resonance structures such as aromatic rings permits the preparation of a compound having particularly high optical anisotropy, while the selection of a ring or rings each including a large number of sites free of any resonance structure such as trans-cyclohexane-1,4-diyl ring permits the preparation of a compound having low optical anisotropy.

The degree of freedom in the design of a liquid crystal panel would widely be expanded by arbitrarily selecting the optical anisotropy value.

All of the compounds according to the first aspect of the present invention and preferred embodiments thereof have low viscosities and therefore, they do not undesirably increase the overall viscosity of the resulting liquid crystal composition even if a large amount of the compound is incorporated into the composition. In addition, the compound of the present invention exhibits very low temperature-dependency of the viscosity, in particular, at a low temperature. The use of the liquid crystal compound having such an excellent viscosity permits the preparation of a liquid crystal composition having high speed responsibility.

All of the compounds according to the first aspect of the present invention and preferred embodiments thereof are physicochemically stable and accordingly, the liquid crystal compositions prepared using them have high specific resistance and a high voltage-holding rate. In other words, the compounds are highly stable against external factors such as ultraviolet rays and heat and have physicochemical stability sufficient for use as ingredients of practically used liquid crystal compositions.

The phase transition temperature of the compounds according to the first aspect of the present invention and preferred embodiments thereof can be controlled by appropriately selecting the number of rings attached to each compound. In other words, it would be sufficient to select a compound carrying not less than 4 rings when it is desired for the achievement of a particularly high clearing point, a compound having 3 rings when it is desired for the achievement of a medium clearing point, and a compound having 2 rings when it is desired for the achievement of a particularly low clearing point.

If describing this more fully, the phase transition temperature of the compounds of Formula (2) according to the present invention can be controlled by appropriately selecting the values of a¹, a², a³, a⁴ and a⁵. More specifically, it would be sufficient to select a compound carrying not less than 4 rings and a¹+a²+a³+a⁴+a⁵ ≧4 when it is desired for the achievement of a particularly high clearing point; a compound having 3 rings and a¹+a²+a³+a⁴+a⁵=3 when it is desired for the achievement of a medium clearing point; and a compound having 2 rings and a¹+a²+a³+a⁴+a⁵=2 when it is desired for the achievement of a particularly low clearing point.

As has been discussed above, the compounds of the first aspect of the present invention and the preferred embodiments thereof have various characteristic properties favorable for use as electro-optical display devices. The use of the compounds of the present invention permits the preparation of novel liquid crystal compositions having good characteristics. In addition, the present invention also permits the production of liquid crystal compounds having desired properties by appropriately designing the structure thereof depending on each particular use as well as liquid crystal composition and liquid crystal display devices using the compounds.

For instance, the compound of the present invention is characterized by having a large negative dielectric anisotropy value, but the compound can not only be used in a liquid crystal composition having a negative dielectric anisotropy value, but also can be used to control the dielectric anisotropy value and other characteristics of a composition having a positive dielectric anisotropy value by the addition of the former to the latter.

If describing this more fully, the compound of the present invention and the liquid crystal composition including the same can be used in various display modes which make use of compounds or compositions having negative dielectric anisotropy values (such as the vertically aligned modes as disclosed in J.P. KOKAI No. Hei 2-176625, IPS mode, ECB mode (for instance, HAN mode or DAP mode), DS mode, GH mode or PC mode), in particular, the vertically aligned modes as disclosed in J.P. KOKAI No. Hei 2-176625 and IPS mode. In addition to the foregoing, the compound and composition of the present invention can also be used for the improvement or control of various characteristics (for instance, dielectric anisotropy, elastic constant, optical anisotropy, viscosity, and/or physicochemical stability) of liquid crystal compositions used for various display modes (such as TN mode, STN mode, or TN-based AM (e.g., TFT or MIM) modes) which make use of compounds or compositions having positive dielectric anisotropy values.

The liquid crystal composition of the present invention will now be detailed below. The liquid crystal composition of the present invention preferably comprises 0.1 to 99.9% by weight of at least one compound as defined in the first aspect of the present invention and the preferred embodiments thereof in order to impart excellent characteristic properties to the composition.

More specifically, the liquid crystal composition of the present invention comprises a compound selected from the group consisting of those represented by Formulas (3) to (13) in an appropriate amount depending on each particular purpose, in addition to the compound of the present invention as a first component.

The following are examples of preferred compounds represented by Formulas (3) to (5) which can be used in the liquid crystal composition of the present invention (in these compounds, R² and Y² are the same as those defined above):

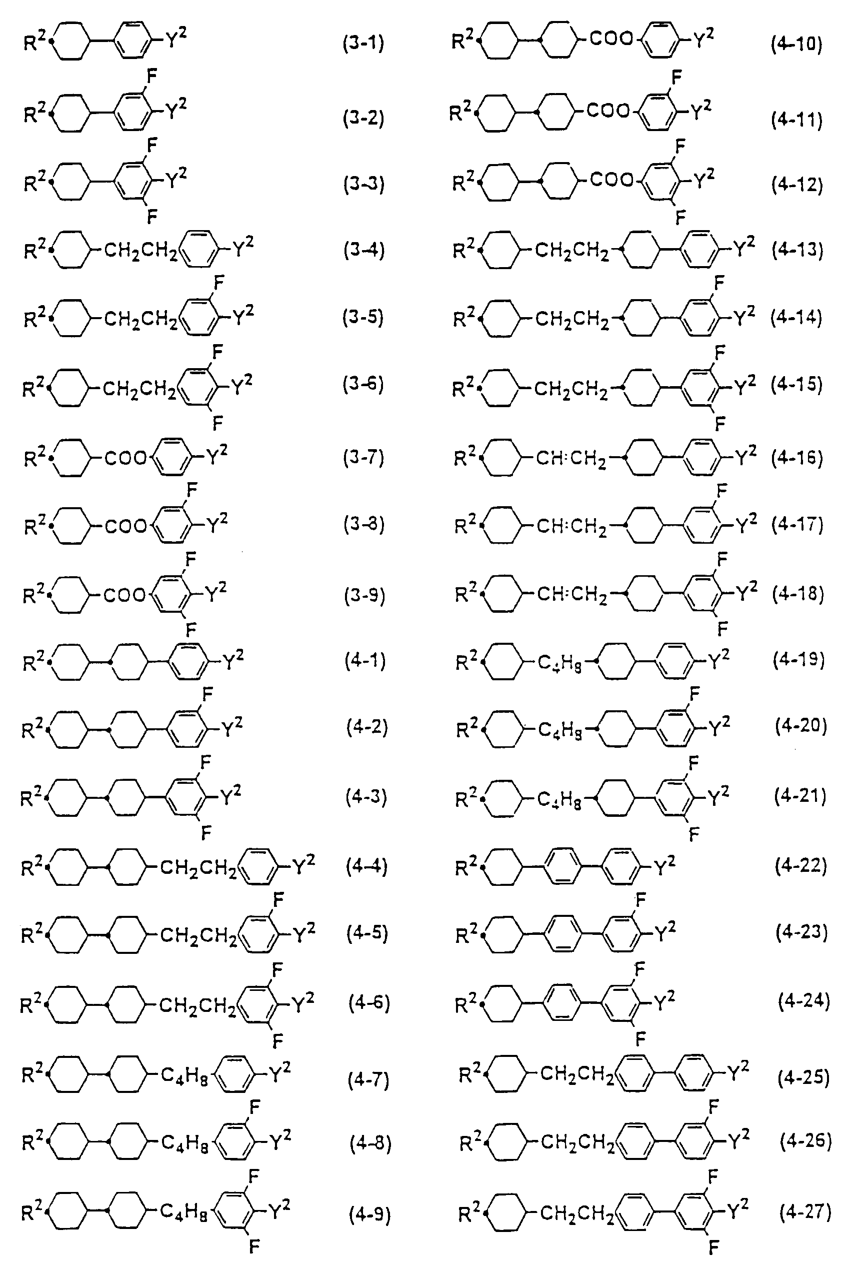

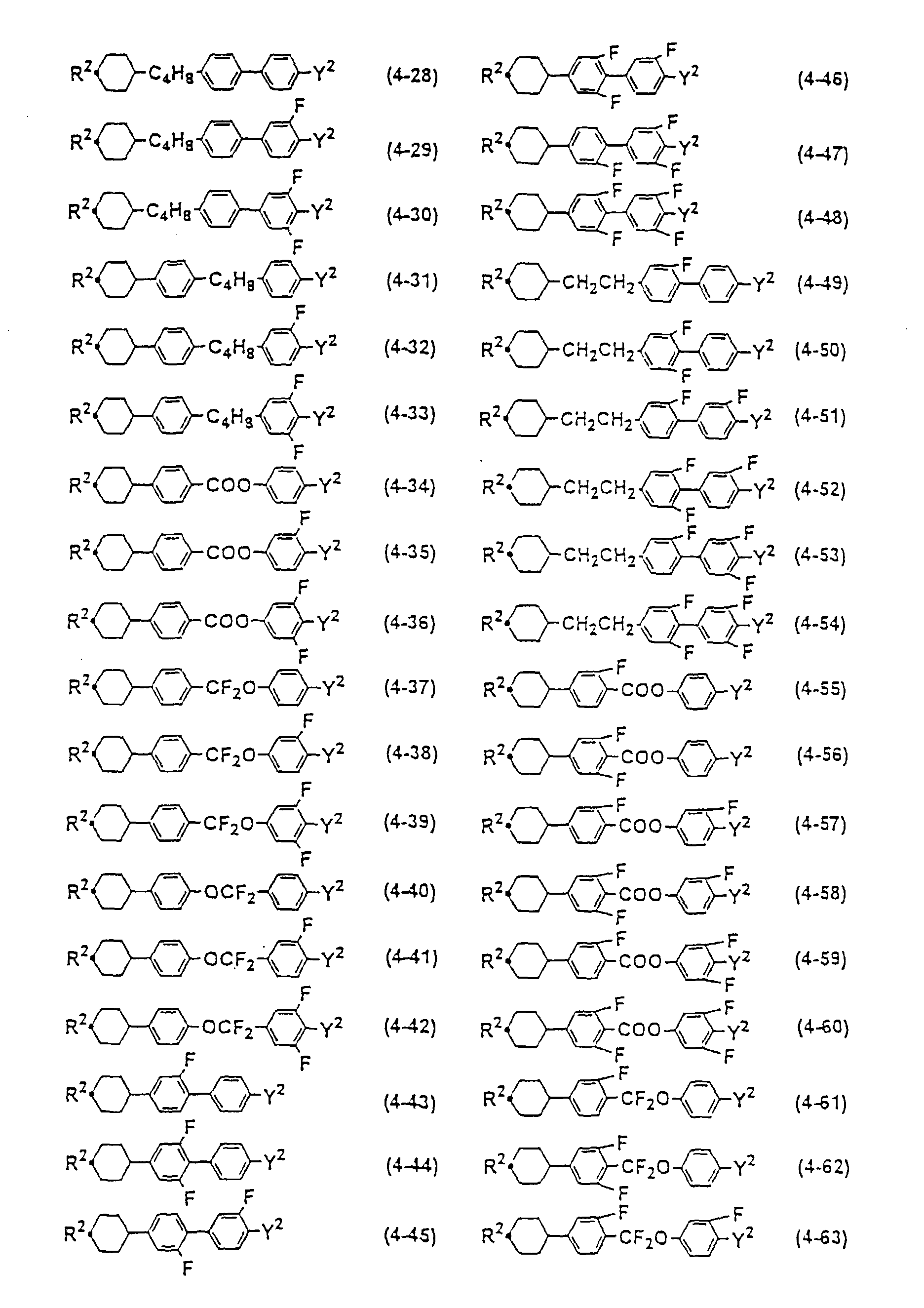

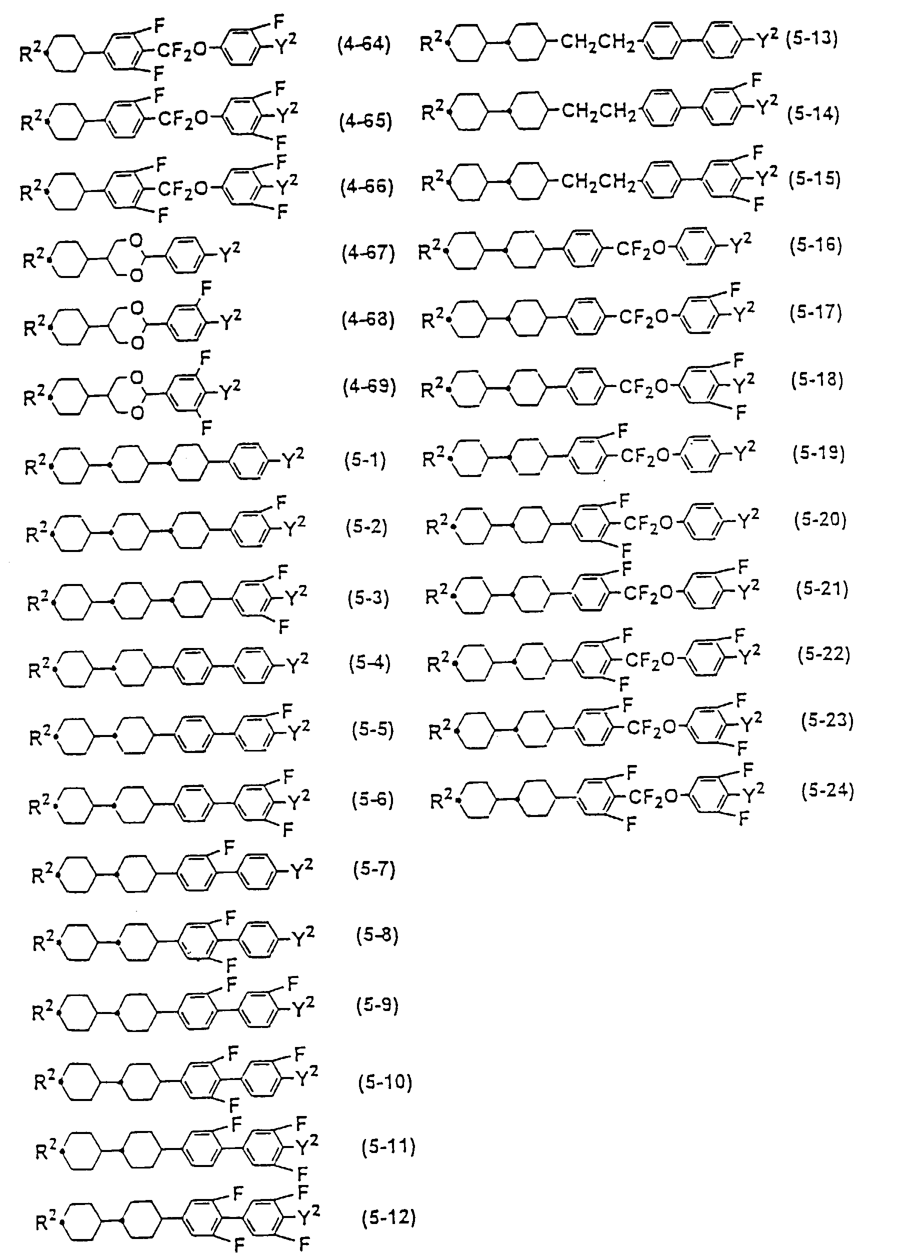

The compounds represented by Formulas (3) to (5) have positive dielectric anisotropy values, are quite excellent in thermal and chemical stability and are particularly preferably used when preparing liquid crystal compositions used in TFT's which require high reliability such as a high voltage holding ratio or a high specific resistance.

The amount of the compounds of Formulas (3) to (5) used when preparing a liquid crystal composition used in TFT's ranges from 0.1 to 99.9% by weight, preferably 10 to 97% by weight and more preferably 40 to 95% by weight based on the total weight of the composition. In addition, the liquid crystal composition may further comprise the compounds of Formulas (8) to (10) in order to control the viscosity of the composition.

The compounds represented by Formulas (3) to (5) can likewise be used when preparing a liquid crystal composition for STN's or TN's. In this case, they are preferably used in an amount of not more than 50% by weight based on the total weight of the composition.

The following are examples of preferred compounds represented by Formulas (6) to (7) which can be used in the liquid crystal composition of the present invention (in these compounds, R³, Y³ and R⁴ are the same as those defined above):

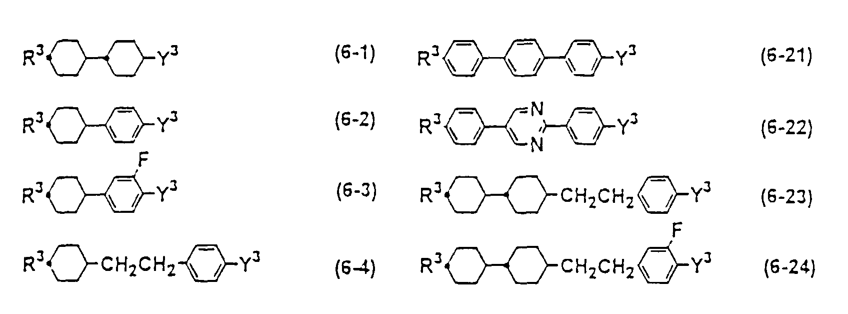

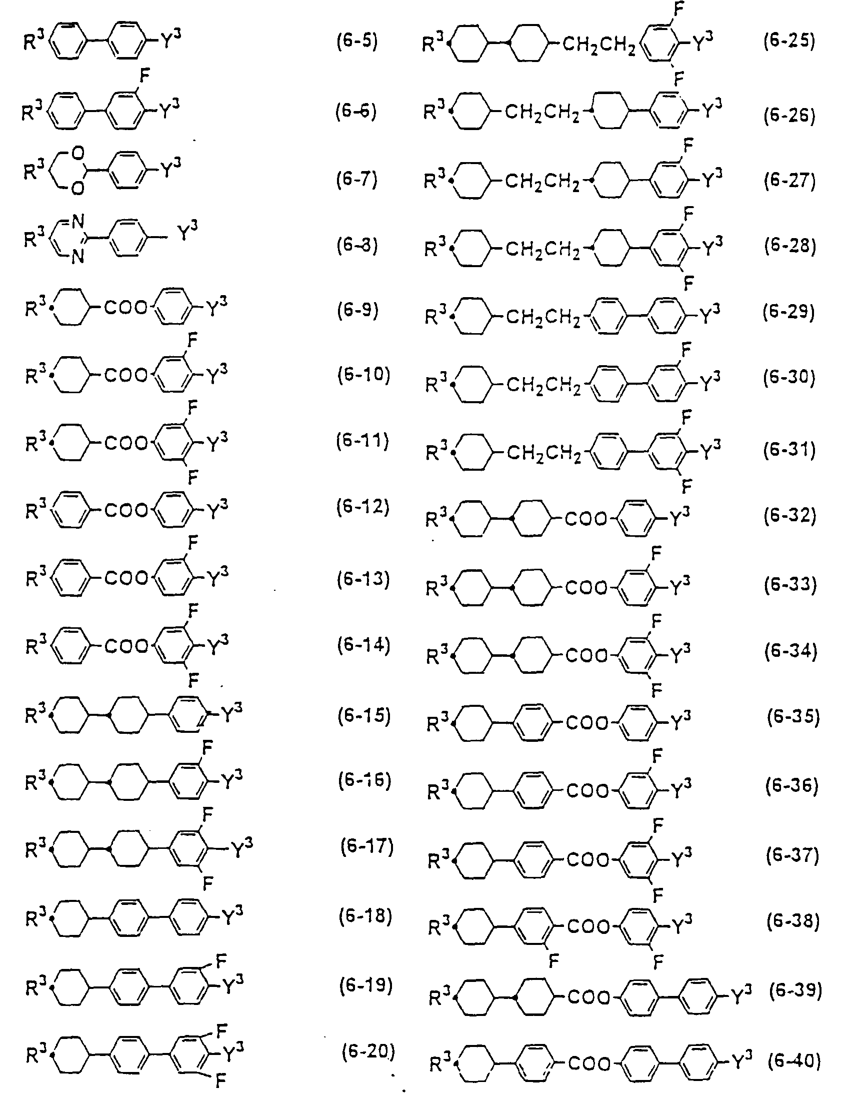

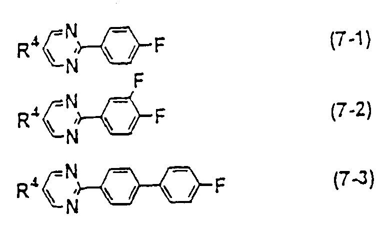

The compounds represented by Formulas (6) to (7) have large and positive dielectric anisotropy values and are used, in particular, for the purpose of reducing the threshold voltage of the resulting liquid crystal composition. Moreover, they can be used for controlling the optical anisotropy value and for expanding the nematic range, for instance, increasing the clearing point. Further they are also used for the improvement in the steepness of the V-T curve of the liquid crystal composition for STN's or TN's.

The compounds represented by Formulas (6) to (7) are preferred, in particular, when preparing the liquid crystal composition for STN's or TN's.

If the amount of the compound of Formula (6) or (7) used increases, the threshold voltage of the resulting liquid crystal composition is reduced and the viscosity thereof increases. Therefore, it is advantageous to use the compounds in a large amount since the resulting composition can be operated at a low voltage, so far as the composition satisfies the requirement for viscosity. If preparing a liquid crystal composition for STN's or TN's, the amount of the compound of Formula (6) or (7) ranges from 0.1 to 99.9% by weight, preferably 10 to 97% by weight and more preferably 40 to 95% by weight based on the total weight of the composition.

The following are examples of preferred compounds represented by Formulas (8) to (10) which can be used in the liquid crystal composition of the present invention (in these compounds, R⁵ and R⁶ are the same as those defined above):

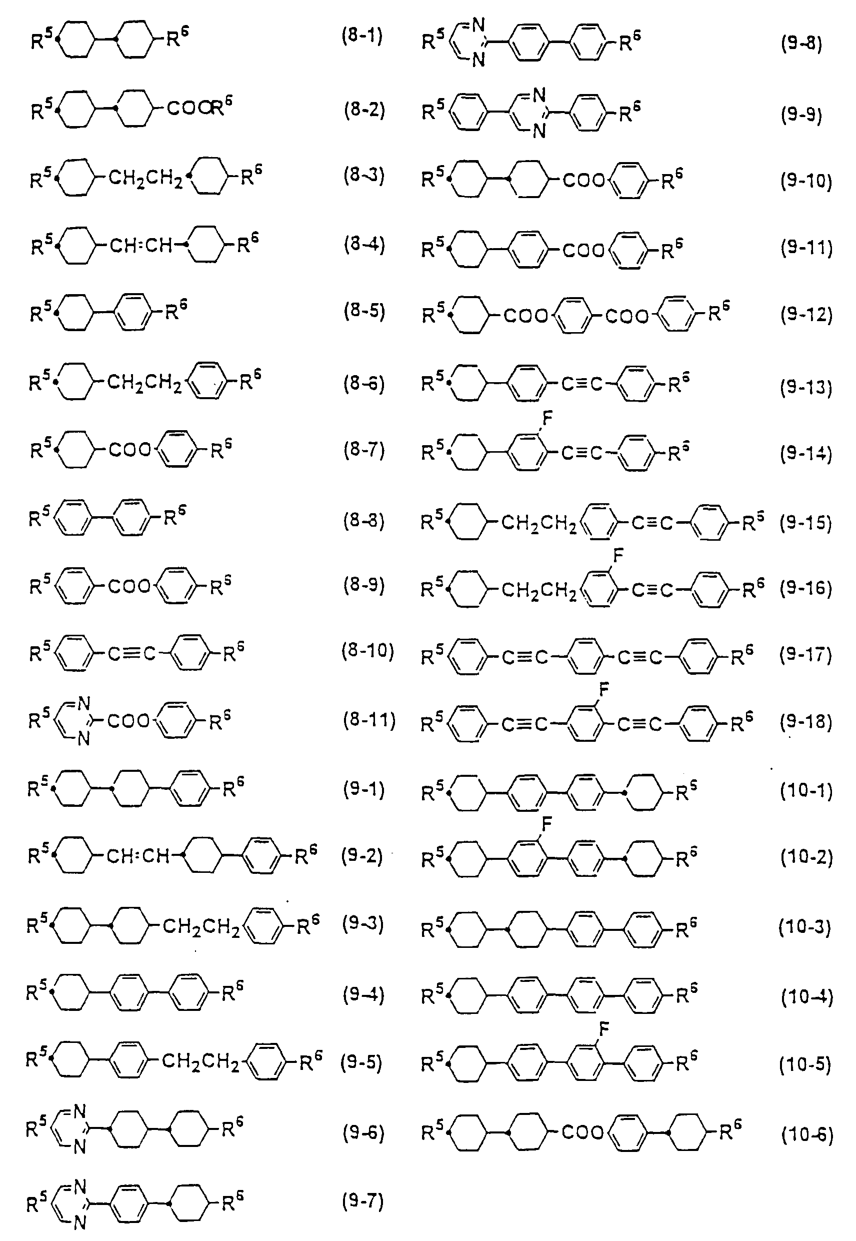

The compounds represented by Formulas (8) to (10) have dielectric anisotropy values whose absolute values are small and are almost neutral ones. The compounds of Formula (8) are mainly used for controlling the viscosity or optical anisotropy value of the resulting liquid crystal composition, while the compounds of Formula (9) and (10) are used for expanding the nematic range, for instance, increasing the clearing point or for controlling the optical anisotropy value.

If the amount of the compounds of Formulas (8) to (10) used increases, the threshold voltage of the resulting liquid crystal composition increases and the viscosity thereof decreases. Therefore, they can be used in a large amount inasmuch as the composition satisfies the requirememt for threshold voltage. When preparing a liquid crystal composition for TFT's, the amount of the compounds of Formulas (8) to (10) is preferably not more than 40% by weight and more preferably not more than 35% by weight based on the total weight of the composition, while when preparing a liquid crystal composition for STN's or TN's, the amount thereof is preferably not more than 70% by weight and more preferably not more than 60% by weight based on the total weight of the composition.

The following are examples of preferred compounds represented by Formulas (11) to (13) which can be used in the liquid crystal composition of the present invention (in these compounds, R⁷ and R⁸ are the same as those defined above):

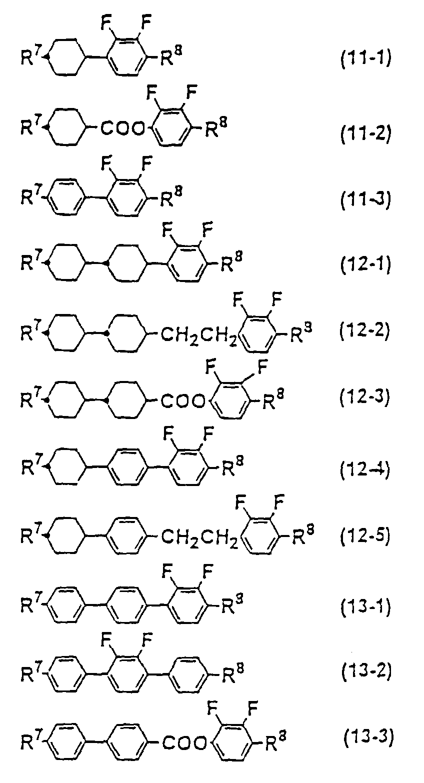

The compounds represented by Formulas (11) to (13) have negative dielectric anisotropy values like the compounds according to the first aspect of the present invention and the preferred embodiments thereof and are used as base compounds for N-type liquid crystal compositions or are used for controlling the dielectric anisotropy value of P-type liquid crystal compositions.

Moreover, the compounds of Formula (11) are bicyclic compounds and mainly used for controlling the threshold voltage, viscosities or optical anisotropy values of the resulting compositions. The compounds of Formula (12) are used for expanding the nematic range, for instance, increasing the clearing point or for controlling the optical anisotropy value of the resulting composition. Further the compounds of Formula (13) are used not only for expanding the nematic range, but also for reducing the threshold voltage and for increasing the optical anisotropy value of the resulting liquid crystal composition.

The compounds of Formula (11) to (13) are mainly used in the N-type liquid crystal compositions and if the amount thereof used increases, the threshold value of the resulting liquid crystal composition is reduced, while the viscosity thereof is increased. For this reason, these compounds are desirably used in a small amount inasmuch as the liquid crystal composition satisfies the requirement for threshold voltage. However, the absolute value of the negative dielectric anisotropy thereof is not more than 5 and therefore, if the amount thereof used is less than 40% by weight, the resulting display device cannot sometimes be operated at a low voltage. When preparing a liquid crystal composition for N-type TFT's, the amount of the compounds of Formula (11) to (13) is preferably not less than 40% by weight and more preferably 50 to 95% by weight based on the total weight of the composition. Moreover, the compounds of Formula (11) to (13) are often incorporated into P-type compositions for controlling the elastic constant and the voltage-transmittance curve (V-T curve) of the resulting liquid crystal composition. In this case, the amount of the compounds of Formula (11) to (13) is preferably not more than 30% by weight based on the total weight of the composition.

In addition, the liquid crystal composition of the present invention may in general comprise an optically active compound in order to induce the formation of a helical structure of the liquid crystal composition to thus achieve a desired twist angle and prevent the occurrence of any reverse twist, except for some particular cases such as liquid crystal compositions for OCB (Optically Compensated Birefringence) mode.

Any known optically active compounds used for such purposes may be used in the present invention, but preferred are optically active compounds listed below:

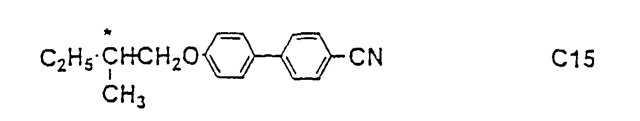

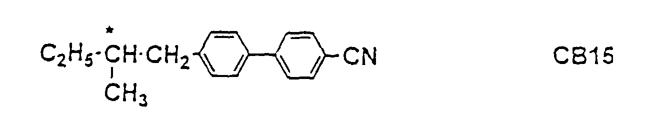

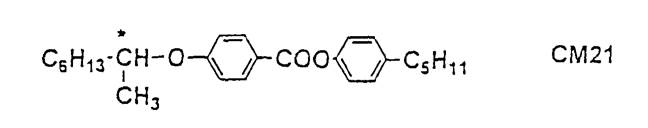

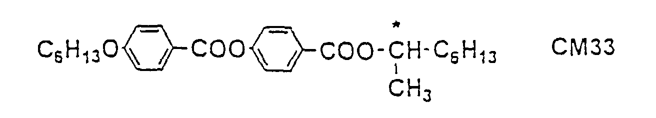

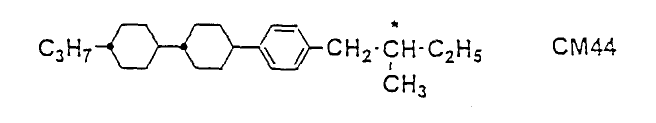

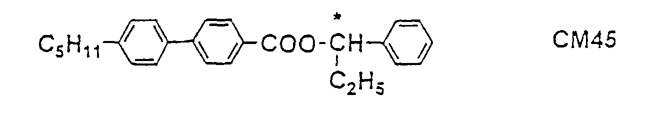

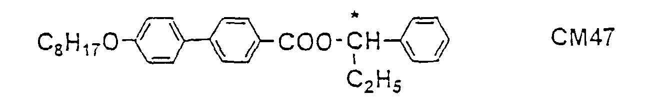

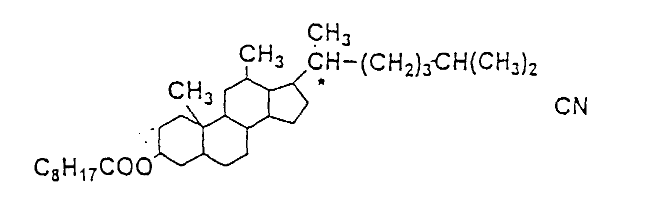

These optically active compounds are usually incorporated into the liquid crystal composition of the present invention to thus control the pitch of twist. The pitch of twist is preferably adjusted to the range of from 40 to 200 µm in case of the liquid crystal compositions for TFT's and TN's. Moreover, it is adjusted to the range of from 6 to 20µm in case of the liquid crystal compositions for STN's and 1.5 to 4 µm for bistable TN's. Moreover, at least two optically active compounds may be added to the composition to control the temperature-dependency of the pitch.

The liquid crystal composition of the present invention may be used as the composition for GH's by addition of a dichroic dye such as merocyanine, styryl, azo, azomethine, azoxy, quinophthalone, anthraquinone, or tetrazine type ones. Alternatively, the composition of the present invention can likewise be used as a liquid crystal composition for polymer-dispersion type liquid crystal display devices (PDLCD) including NCAP's which are produced by encapsulation of a nematic liquid crystal and polymer network liquid crystal display devices (PNLCD) produced by forming a three-dimensional network polymer in the liquid crystal. Moreover, the liquid crystal composition of the present invention may be used in DS's.

The composition of the present invention can be prepared by the commonly used methods. In general, it is prepared by a method wherein various ingredients are dissolved together at a high temperature.

The compounds according to the first aspect of the present invention and the preferred embodiments thereof can easily be prepared by any known organic chemical synthesis technique. An example of the production methods will be given in the following reaction scheme 1:

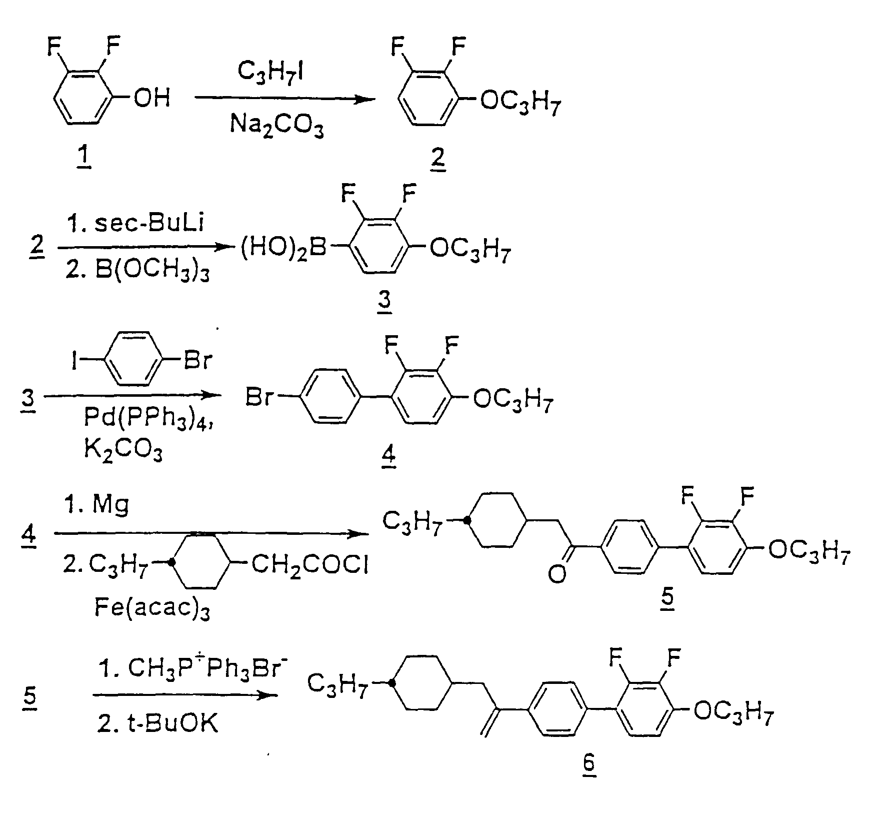

The foregoing route of synthesis will be detailed below.

The compound represented by Formula 1 is reacted with n-propyl iodide in the presence of sodium carbonate to give a compound represented by Formula 2.

The compound represented by Formula 2 is converted into a lithium derivative with sec-butyl lithium and then reacted with trimethyl borate to give a compound of Formula 3.

The compound represented by Formula 3 is reacted with 4-bromoiodobenzene in the presence of tetrakis(triphenyl phosphine)palladium (0) and sodium carbonate to give a compound of Formula 4.

The compound represented by Formula 4 is reacted with magnesium to give a Grignard reagent and then reacted with trans-4-n-propylcyclohexyl acetyl chloride in the presence of iron(3) acetylacetonate to give a compound of Formula 5.

The compound represented by Formula 5 is reacted with an ylide obtained through the reaction of methyltriphenyl phosphonium bromide with potassium t-butoxide to give a desired compound of Formula 6.

The method of preparing the compound of the present invention and the use thereof will be described below in detail with reference to the following working Examples, but the present invention is not restricted to these specific Examples at all. In the description of the following Examples, the symbol N represents the normal concentration (gram eq./1); M represents the molar concentration (mole/l); Δ ε(dielectric anisotropy value) of a liquid crystal compound represents the value obtained by extrapolating the values (as measured at 25 °C) observed for the compositions comprising 85 wt% of the following mother liquid crystal A and 15 wt% of each corresponding compound; and Δn (optical anisotropy value) represents the measured value (as determined at 25 °C) for the composition comprising 85 wt% of the following mother liquid crystal B and 15 wt% of each corresponding compound.

### Composition of Mother Liquid Crystal A

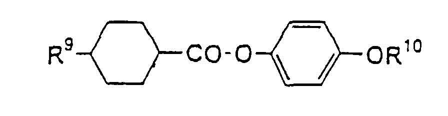

Five kinds of ester compounds represented by the foregoing general formula and whose both terminal alkyl groups (R⁹, R¹⁰) were different from each other were mixed in a ratio specified below to give the desired mother liquid crystal A.

| | | |
|---|---|---|
| R⁹ = C₃H₇, | R¹⁰ = C₄H₉ | 27.6 wt% |
| R⁹ = C₄H₉, | R¹⁰ = C₂H₅ | 20.7 wt% |
| R⁹ = C₅H₁₁, | R¹⁰ = CH₃ | 20.7 wt% |
| R⁹ = C₃H₇, | R¹⁰ = C₂H₅ | 17.2 wt% |
| R⁹ = C₅H₁₁, | R¹⁰ = C₂H₅ | 13.8 wt% |
| | | 100.0 |
| Δ ε = -1.5 | | |

### Composition of Mother Liquid Crystal B

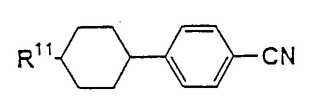

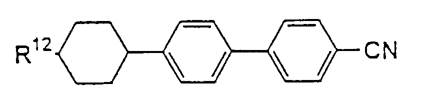

Four kinds of compounds represented by the foregoing general formulas and whose both terminal alkyl groups (R¹¹, R¹²) were different from each other were mixed in a ratio specified below to give the desired mother liquid crystal B.

| | |
|---|---|
| R¹¹ = C₃H₇ | 24.0 wt% |
| R¹¹ = C₅H₁₁ | 36.0 wt% |
| R¹¹ = C₇H₁₅ | 25.0 wt% |
| R¹² = C₅H₁₁ | 15.0 wt% |
| | 100.0 |
| Δ n = 0.137 | |

### Example 1: Synthesis of 2-(2,3-Difluoro-4-n-Propyloxybiphenyl-4'-yl)-3-(Trans-4-n-Propylcyclohexyl)Propene (6)

### First Step: Synthesis of 1,2-Difluoro-3-n-Propyloxybenzene (2)

A mixture of 2,3-difluorophenol (1) (130.1g, 1.00 mole), propyl iodide (255.0g, 1.50 mole), sodium carbonate (212.0g, 2.00 mole) and ethanol (1200 ml) was reacted under reflux for 6 hours, in a nitrogen gas atmosphere. After the completion of the reaction, the reaction solution was cooled to room temperature and then poured into a 1N dilute hydrochloric acid solution (1500 ml) with cooling. The insoluble matter formed was removed by filtration. The resulting filtrate was extracted with toluene (2000 ml), followed by washing the resulting oprganic phase with water (1000 ml) and drying over anhydrous magnesium sulfate. The solvent was distilled off under reduced pressure, the resulting residue was purified by silica gel column chromatography (eluent: hexane) to give the title compound, 1,2-difluoro-3-n-propyloxybenzene (2)(158.4g, 0.921 mole; yield 92%). MS/172 (M ⁺ ).

### Second Step: Synthesis of 2,3-Difluoro-4-Propyloxyphenyl Boric Acid (3)

To a mixture of 1,2-difluoro-3-n-propyloxybenzene (2) (158.2g, 0.919 mole) prepared in the first step and THF (tetrahydrofuran) (500 ml), there was dropwise added, at -78°C, a solution of 1.56M sec-butyl lithium in hexane (0.921 mole, 590 ml) over 30 minutes and the reaction was continued as it was for one hour. At that temperature, a solution of trimethyl borate (144.5g, 1.39 mole) in THF (300 ml) was dropwise added to the reaction solution over 30 minutes, then the temperature thereof was gradually raised and they were reacted at 0 °C for 3 hours. After completion of the reaction, the reaction solution was added to a 2N dilute hydrochloric acid solution (1000 ml) followed by extraction with diethyl ether (1000 ml). The resulting oprganic phase was washed with water (800 ml) and dried over anhydrous magnesium sulfate. The solvent was distilled off under reduced pressure and the resulting residue was washed with hexane to give the title compound, 2,3-difluoro-4-propyloxyphenyl boric acid (3) (122.1g, 0.565 mole; Yield 62%). MS/216 (M ⁺ )

### Third Step: Synthesis of 2,3-Difluoro-4-Propyloxy-4'-Bromobiphenyl (4)

There were mixed 2,3-difluoro-4-propyloxyphenyl boric acid (3) (121.9g, 0.564 mole) prepared in the second step, 4-bromoiodobenzene (177.4g, 0.627 mole), tetrakis(triphenyl phosphine)palladium (0) (32.59g, 0.0282 mole), potassium carbonate (236.3g, 1.71 mole) and a toluene-ethanol-water (mixing ratio 7:7:0.5) mixed solvent (1200 ml), followed by refluxing the mixture with heating for 4 hours. After completion of the reaction, water (1000 ml) was added to the reaction mixture and then the mixture was extracted with toluene (1800 ml). The resulting organic phase was dried over anhydrous magnesium sulfate. The solvent was distilled off under reduced pressure, the resulting residue was purified by silica gel column chromatography (eluent: hexane) to give the title compound, 2,3-difluoro-4-propyloxy-4'-bromobiphenyl (4) (46.72g, 0.143 mole; yield 25%). MS/327 (M ⁺ ).

### Fourth Step: Synthesis of 2,3-Difluoro-4-n-Propyloxy-4'-(1-Oxo-2-(Trans-4-n-Propylcyclohexyl)Ethyl)Biphenyl (5)

A mixture of 2,3-difluoro-4-propyloxy-4'-bromobiphenyl (4) (46.58g, 0.142 mole), magnesium (3.63g, 0.149 mole) and THF (120 ml) was reacted in a sealed tube at 150 °C for 30 minutes to give a Grignard reagent.

In a separate container, there were mixed trans-4-n-propylcyclohexyl acetyl chloride (43.29g, 0.214 mole), iron(3) acetylacetonate (5.03g, 0.0142 mole) and toluene (500 ml) and then the resulting mixture was cooled to -40°C. To the mixture, there was dropwise added the Grignard reagent prepared above while maintaining the mixture at that temperature, followed by the reaction at that temperature for 2 hours. After completion of the reaction, a 1N dilute hydrochloric acid solution (300 ml) was dropwise added to the reaction solution with ice-cooling and then the reaction solution was extracted with toluene (300 ml). The resulting organic phase was washed with water (300 ml) and then dried over anhydrous magnesium sulfate. The solvent was distilled off under reduced pressure, the resulting residue was purified by silica gel column chromatography (eluent: hexane) to give the title compound, 2,3-difluoro-4-n-propyloxy-4'-(1-oxo-2-(trans-4-n-propylcyclohexyl)ethyl)biphenyl (5) (33.76g, 0.0814 mole; yield 57%). MS/415 (M ⁺ ).

### Fifth Step: Synthesis of 2-(2,3-Difluoro-4-n-Propyloxybiphenyl-4'-yl)-3-(Trans-4-n-Propylcyclohexyl)Propene (6)

To a solution of methyltriphenyl phosphonium chloride (43.46g, 0.122 mole) in THF (100 ml), there was dropwise added, with ice-cooling, a solution of potassium t-butoxide (13.65g, 0.122 mole) in THF (50 ml) and then they were reacted at room temperature for one hour. Thereafter, to the solution, there was dropwise added a solution of 2,3-difluoro-4-n-propyloxy-4'-(1-oxo-2-(trans-4-n-propylcyclohexyl) ethyl)biphenyl (5) (33.62g, 0.0811 mole) prepared in the 4th step in THF (100 ml) over 30 minutes with ice-cooling, and the reaction was continued at room temperature overnight. After completion of the reaction, water (150 ml) was added to the reaction solution and then the latter was extracted with toluene (500 ml). The resulting organic phase was washed with water (200 ml) and then dried over anhydrous magnesium sulfate. The solvent was distilled off under reduced pressure, the resulting residue was purified by silica gel column chromatography (eluent: hexane) and then recrystallization (ethanol) to give the title compound, 2-(2,3-difluoro-4-n-propyloxybiphenyl -4'-yl)-3-(trans-4-n-propylcyclohexyl)propene (6) (23.72g, 0.0575 mole; yield 71%). MS/413 (M ⁺ ); Δ ε= -3.5; Δ n=0.148.

The following compounds can be prepared according to the method described in Example 1.

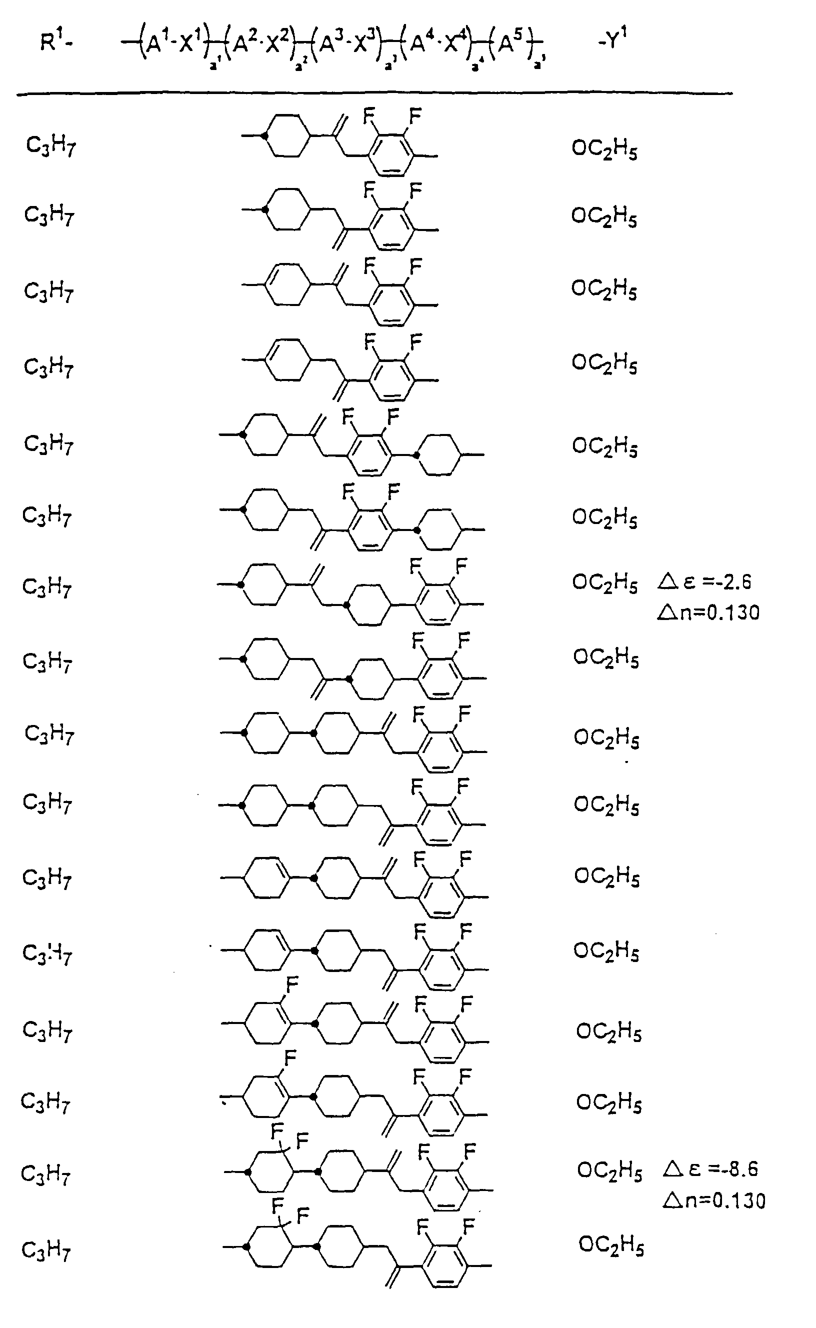

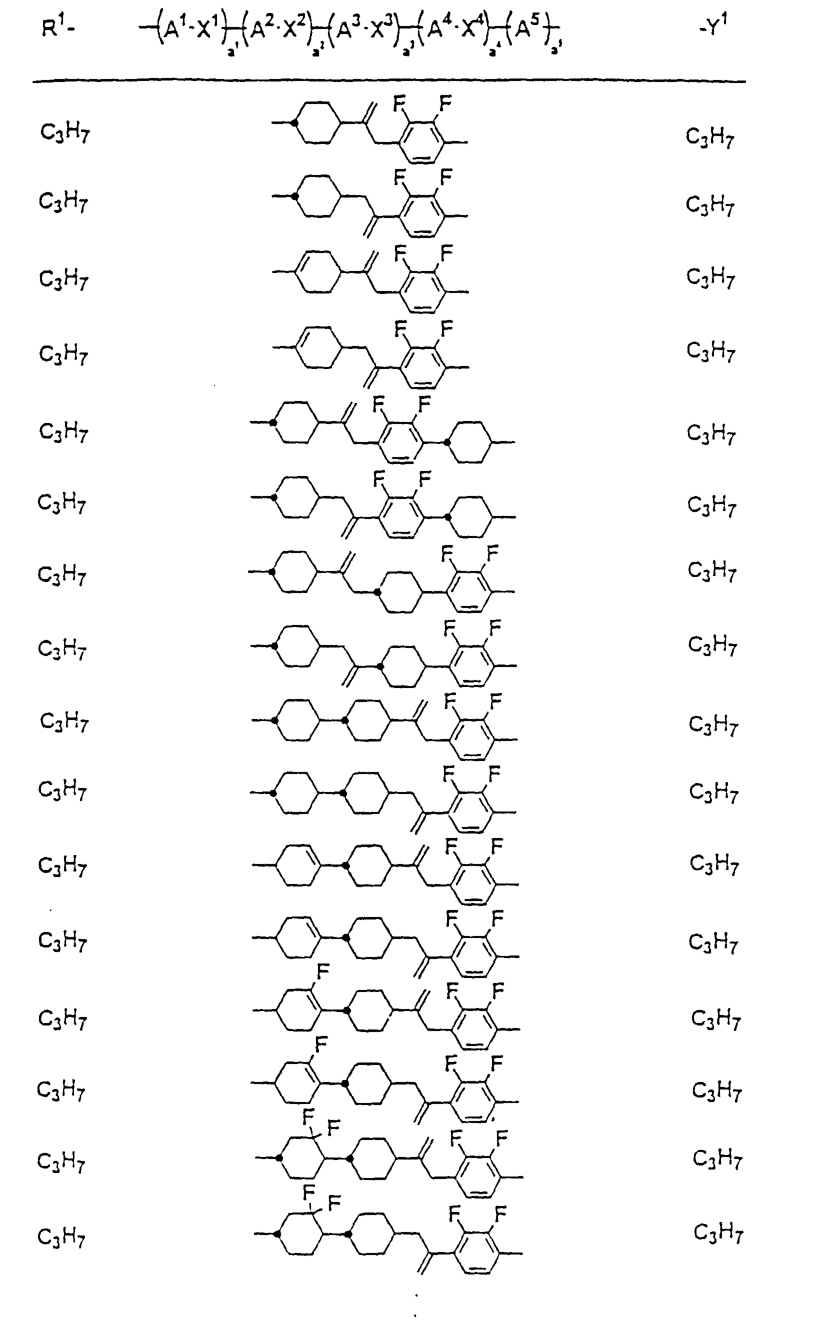

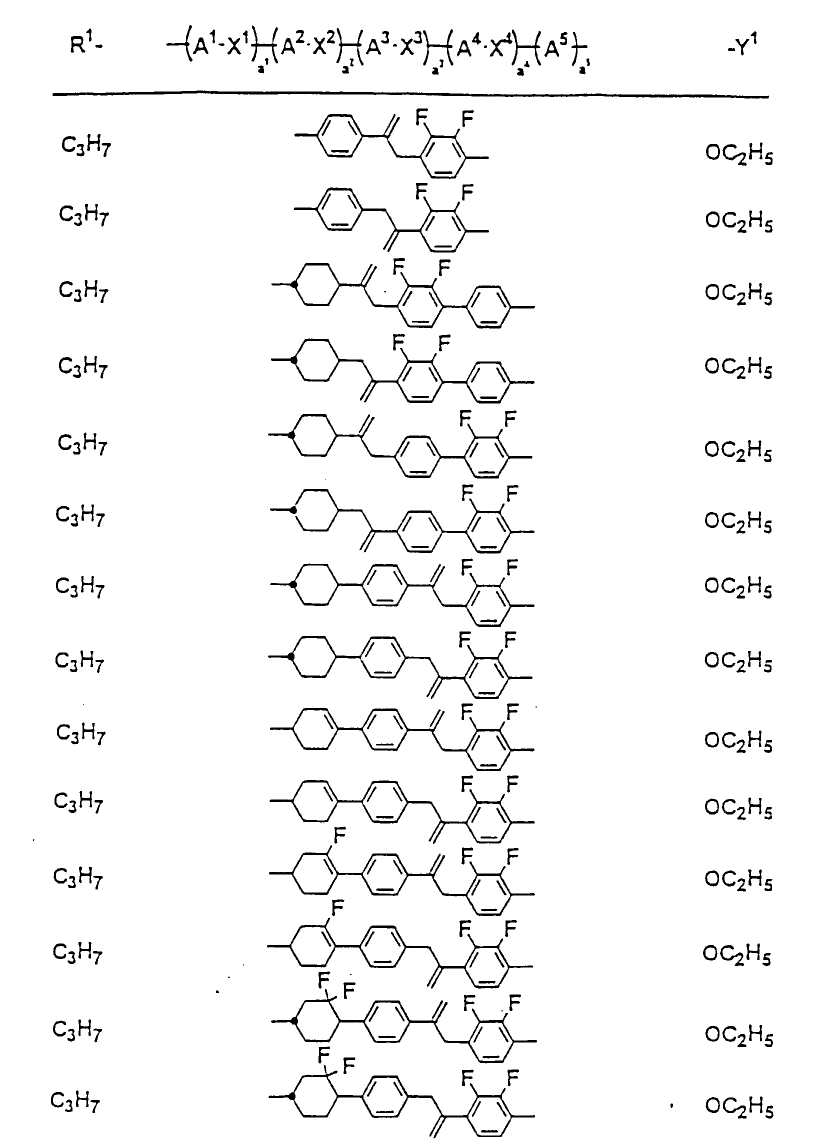

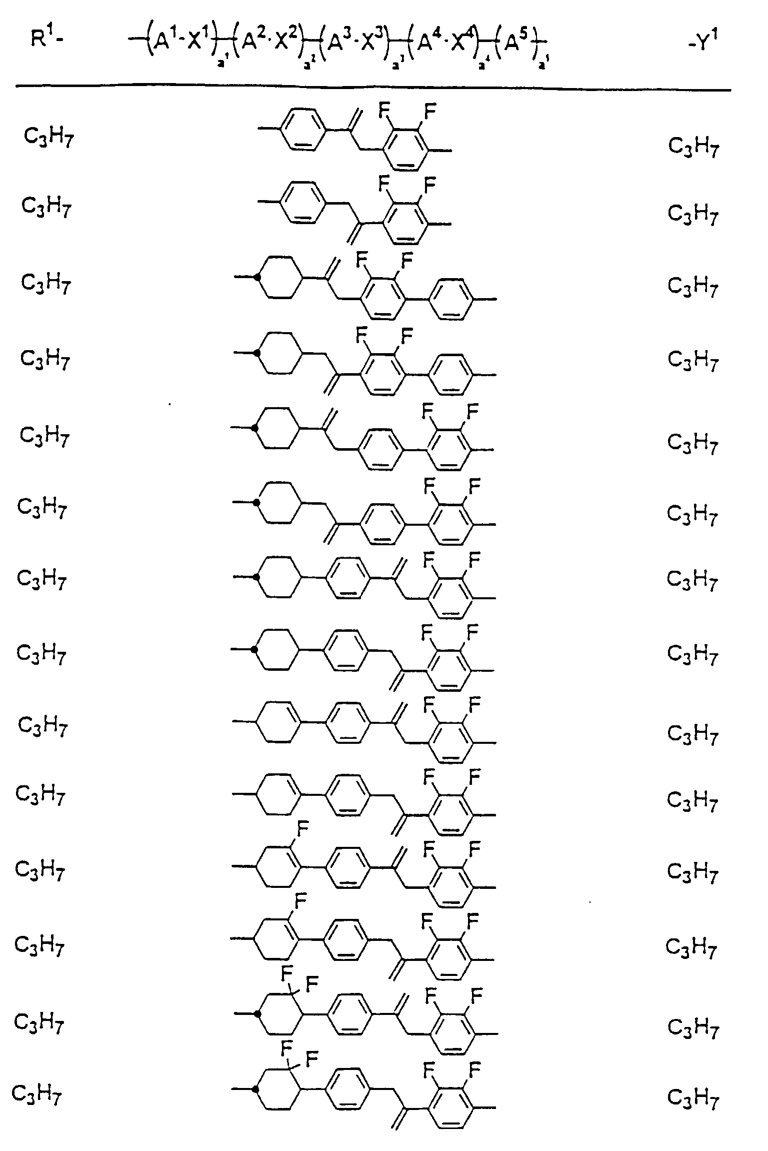

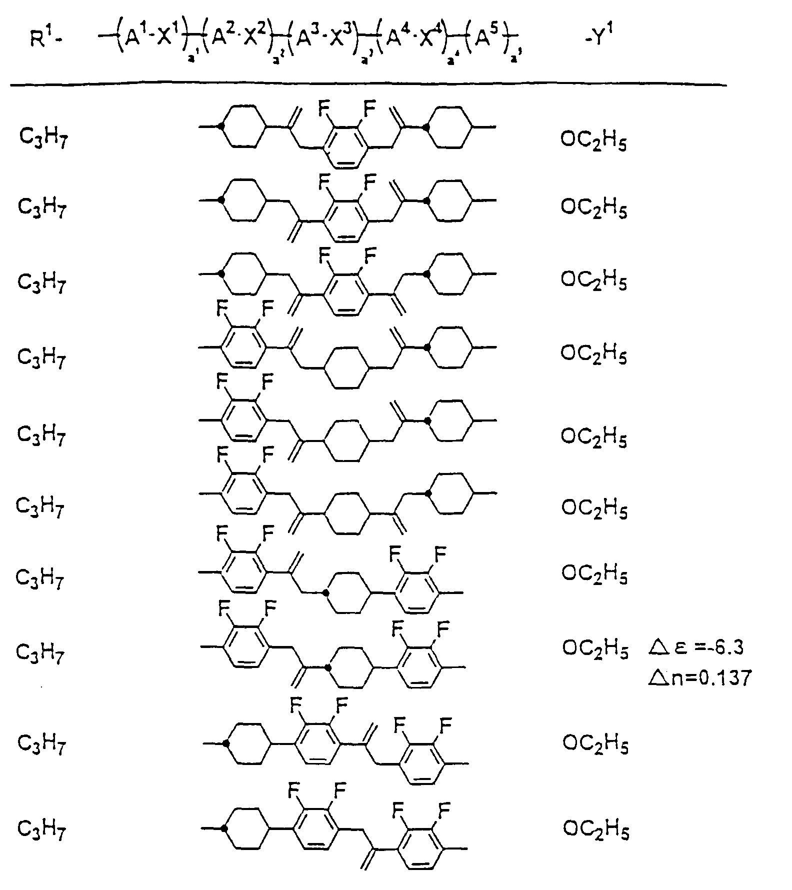

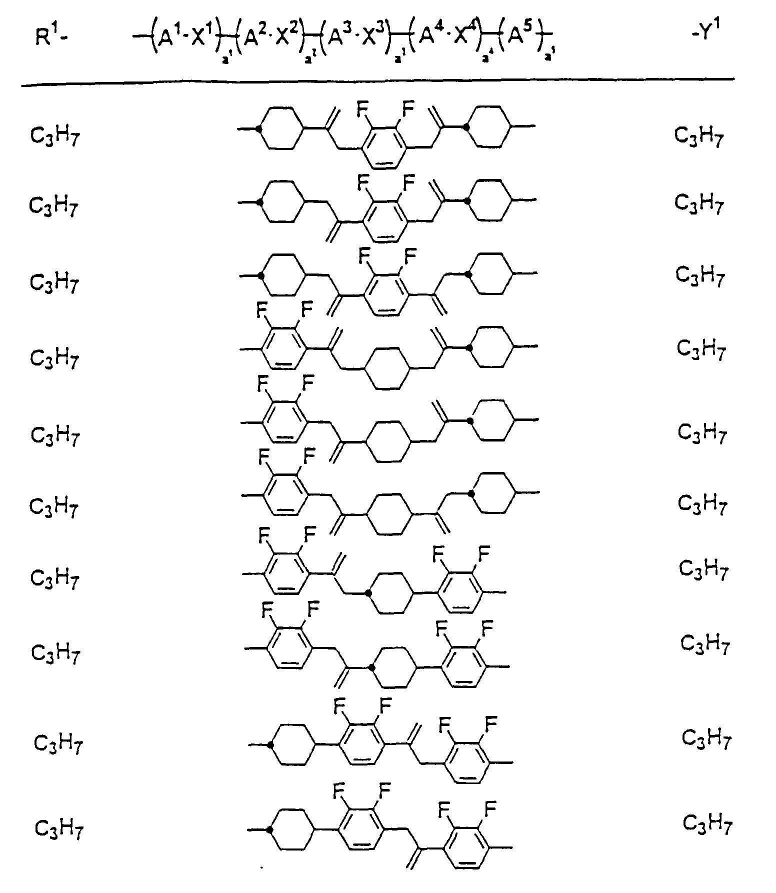

As has been described above in detail, the present invention can provide a liquid crystal compound which has a negative dielectric anisotropy value whose absolute value is large, a low viscosity, a controlled optical anisotropy value, a high specific resistance and a high voltage holding ratio and which is stable against heat and irradiation with ultraviolet rays, as well as a liquid crystal composition containing the compound and a liquid crystal display device produced using the composition.

## Claims

1. A liquid crystal compound having -C(=CH₂)CH₂- and/or -CH₂C(=CH₂)- groups and a ring represented by the following general formula (1) in the general structure of the compound: 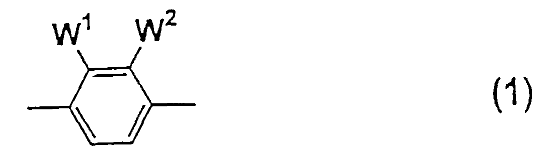 wherein W¹ and W² each independently represents a fluorine or chlorine atom, provided that each atom constituting the compound may be substituted with its isotope.

2. The compound of claim 1 represented by the following general formula (2):
R¹-(A¹-X¹)ₐ₁-(A²-X²)ₐ₂-(A³-X³)ₐ₃-(A⁴-X⁴)ₐ₄-(A⁵)ₐ₅-Y¹ (2)
wherein R¹ and Y¹ each independently represents an alkyl group having 1 to 20 carbon atoms, a hydrogen atom, a halogen atom, a cyano group, a cyanate residue, an isocyano group or an isothiocyanate residue, provided that at least one of the methylene groups present in the alkyl group, which are not adjacent to one another, may be substituted with an oxygen, sulfur or nitrogen atom, -C ≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom or that these alkyl groups may be optically active ones; X¹, X², X³ and X⁴ each independently represents a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡C-, -C≡CCH=CH-, -C(=CH₂)CH₂- or -CH₂C(=CH₂)-, provided that at least one of the substituents X¹, X², X³ and X⁴ represent a group -C(=CH₂)CH₂- or -CH₂C(=CH₂)-; the rings A¹, A², A³, A⁴ and A⁵ each independently represents a trans-cyclohexane-l,4-diyl, cyclohexane-1-en-1,4-diyl, 1,4-phenylene, bicyclo[1.1.1] pentane-1,3-diyl group, or a ring represented by the following formula (1): 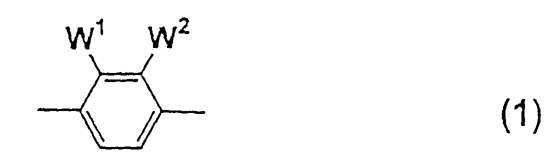 wherein W¹ and W² each independently represents a fluorine or chlorine atom, provided that at least one of these rings A¹, A², A³, A⁴ and A⁵ represent a ring represented by Formula (1), that the carbon atoms constituting these rings may be substituted with a nitrogen, oxygen or sulfur atom and that each hydrogen atom present on the rings may be substituted with a halogen atom or a cyano group; a¹, a², a³, a⁴ and a⁵ each independently represents 0 or 1, provided that they satisfy the relation: a¹+a²+a³+a⁴+a⁵ ≧ 1; and each atom constituting the compound may be substituted with its isotope.

3. The compound of claim 1 wherein the group represented by Formula (1) is 2,3-difluorobenzene-1,4-diyl.

4. A liquid crystal composition comprising at least two components including at least one liquid crystal compound selected from the group consisting of those set forth in any one of claims 1 to 3.

5. A liquid crystal composition comprising at least one liquid crystal compound selected from the group consisting of those set forth in any one of claims 1 to 3 as a first component and at least one compound selected from the group consisting of those represented by the following general formulas (3), (4) and (5): 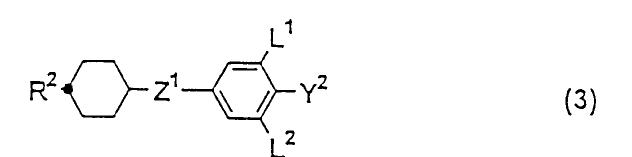 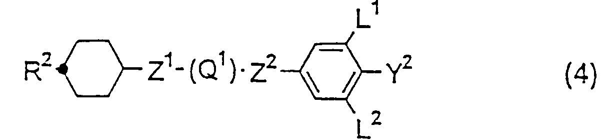 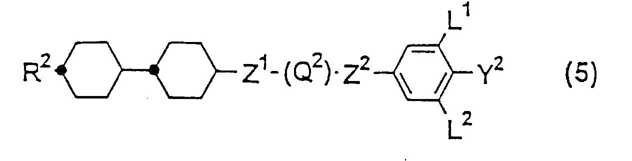 wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom, -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents -(CH₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-l,4-diyl group, or a 1,4-phenylene group whose hydrogen atom may be substituted with a fluorine atom; and each atom constituting the compound may be substituted with its isotope.

6. A liquid crystal composition comprising at least one liquid crystal compound selected from the group consisting of those set forth in any one of claims 1 to 3 as a first component and at least one compound selected from the group consisting of those represented by the following general formulas (6) and (7) as a second component: 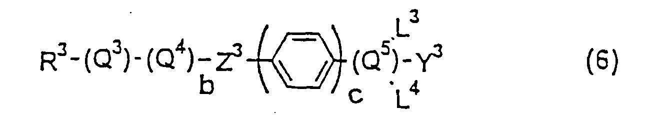 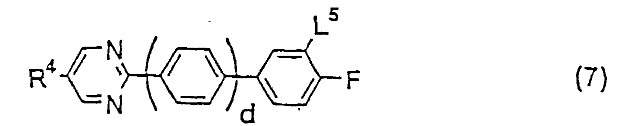 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cyano group or -C ≡C-CN; the ring Q³ represents a trans-cyclohexane-1,4-diyl, 1,4-phenylene, 1,3-dioxane-2,5-diyl or pyrimidine-2,5-diyl group; the ring Q⁴ represents a trans-cyclohexane-1,4-diyl or pyrimidine-2,5-diyl group, or a 1,4-phenylene group whose hydrogen atom may be substituted with a fluorine atom; the ring Q⁵ represents a trans-cyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

7. A liquid crystal composition comprising at least one liquid crystal compound selected from the group consisting of those set forth in any one of claims 1 to 3 as a first component; at least one compound selected from the group consisting of those represented by the foregoing general formulas (3), (4) and (5) as a second component; and at least one compound selected from the group consisting of those represented by the following general formulas (8), (9) and (10) as a third component:
R⁵·(Q⁶)-Z⁴·(Q⁷)-Z⁵―R⁶ (8)
R⁵·(Q⁶)-Z⁴·(Z⁷)-Z⁵-(Q⁸)·R⁶ (9) 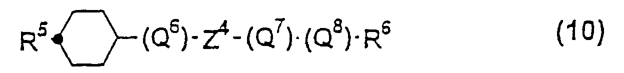
wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1,4-phenylene group whose hydrogen atom may be substituted with a fluorine atom; Z⁴ and Z⁵ each independently represents -C≡C-, -COO-, -(CH₂)₂-, -CH=CH- or a single bond; and each atom constituting the compound may be substituted with its isotope.

8. A liquid crystal composition comprising at least one liquid crystal compound selected from the group consisting of those set forth in any one of claims 1 to 3 as a first component and at least one compound selected from the group consisting of those represented by the following general formulas (11), (12) and (13) as a second component: 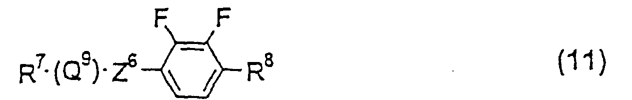 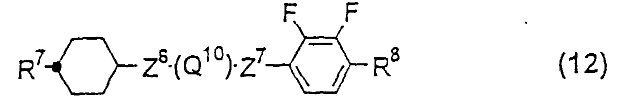 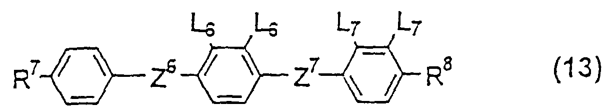 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁶ and L⁷ do not simultaneously represent a hydrogen atom; Z⁶ and Z⁷ each independently represents -(CH₂)₂- -COO- or a single bond; and each atom constituting the compound may be substituted with its isotope.

9. A liquid crystal composition comprising at least one liquid crystal compound selected from the group consisting of those set forth in any one of claims 1 to 3 as a first component; at least one compound selected from the group consisting of those represented by the foregoing general formulas (8), (9) and (10) as a second component; and at least one compound selected from the group consisting of those represented by the foregoing general formulas (11), (12) and (13) as a third component.

10. A liquid crystal composition comprising at least one liquid crystal compound selected from the group consisting of those set forth in any one of claims 1 to 3 as a first component; at least one compound selected from the group consisting of those represented by the foregoing general formulas (6) and (7) as a second component; and at least one compound selected from the group consisting of those represented by the foregoing general formulas (8), (9) and (10) as a third component.

11. A liquid crystal composition comprising at least one liquid crystal compound selected from the group consisting of those set forth in any one of claims 1 to 3 as a first component; at least one compound selected from the group consisting of those represented by the foregoing general formulas (3), (4) and (5) as a second component; at least one compound selected from the group consisting of those represented by the foregoing general formulas (6) and (7) as a third component; and at least one compound selected from the group consisting of those represented by the foregoing general formulas (8), (9) and (10) as a fourth component.

12. A liquid crystal composition comprising at least one optically active compound in addition to a liquid crystal composition as set forth in any one of claims 4 to 11.

13. A liquid crystal display device constituted by the use of a liquid crystal composition as set forth in any one of claims 4 to 12.
